(19) Europäisches Patentamt
European Patent Office
Office européen des brevets

(11) EP 2 727 913 A1

(12) **EUROPEAN PATENT APPLICATION**

(43) Date of publication:
**07.05.2014 Bulletin 2014/19**

(21) Application number: **14153398.4**

(22) Date of filing: **14.12.2009**

(51) Int Cl.:
*C07D 241/04* (2006.01)
*C07D 417/12* (2006.01)
*A61K 31/496* (2006.01)
*A61P 25/00* (2006.01)

(84) Designated Contracting States:
**AT BE BG CH CY CZ DE DK EE ES FI FR GB GR HR HU IE IS IT LI LT LU LV MC MK MT NL NO PL PT RO SE SI SK SM TR**
Designated Extension States:
**AL BA RS**

(30) Priority: **15.12.2008 US 122445**

(62) Document number(s) of the earlier application(s) in accordance with Art. 76 EPC:
**09833731.4 / 2 379 519**

(71) Applicant: **Astrazeneca AB**
**SE-151 85 Södertälje (SE)**

(72) Inventors:
- **Cumming, John**
  **Macclesfield, Cheshire SK10 4TG (GB)**
- **Faull, Alan, Wellington**
  **Macclesfield, Cheshire SK10 4TG (GB)**
- **Waterson, David**
  **Macclesfield, Cheshire SK10 4TG (GB)**

(74) Representative: **Gainey, Laurence David Scott**
**AstraZeneca PLC**
**Global Intellectual Property**
**Mereside**
**Alderley Park**
**Macclesfield, Cheshire SK10 4TG (GB)**

Remarks:
This application was filed on 31-01-2014 as a divisional application to the application mentioned under INID code 62.

(54) **(4-Tert-butylpiperazin-2-yl)(piperazin-1-yl)methanone-N-carboxamide derivatives**

(57) The present invention relates to compounds of formula (I)

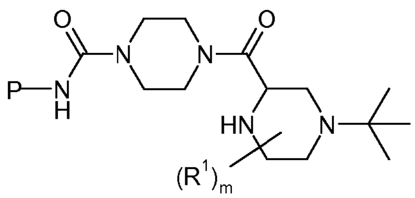

(I)

The compounds act via antagonism of the CCR2b receptor and may be used to treat inflammatory disease and/or neuropathic pain.

EP 2 727 913 A1

## Description

The present invention relates to pharmaceutical compositions, which comprise compounds that act via antagonism of the CCR2b receptor for which MCP-1 is one of the known ligands and so may be used to treat inflammatory disease and/or neuropathic pain, which is mediated by these receptors. The invention further relates to novel compounds for use in the compositions, to processes for their preparation, to intermediates useful in their preparation and to their use as therapeutic agents.

Chemokines play an important role in immune and inflammatory responses in various diseases and disorders, including rheumatoid arthritis, chronic obstructive pulmonary disease, atherosclerosis and other autoimmune pathologies such as inflammatory bowel disease, diabetes, asthma and allergic diseases. Chemokines also have a role in neuropathic pain, angiogenesis and modulation of chemokines may be beneficial in the treatment of cancer. Chemokines are small secreted molecules belonging to a growing superfamily of 8-14 kDa proteins characterized by a conserved four cysteine motif. The chemokine superfamily can be divided into two main groups exhibiting characteristic structural motifs, the Cys-X-Cys (C-X-C) and Cys-Cys (C-C) families. These are distinguished on the basis of a single amino acid insertion between the NH-proximal pair of cysteine residues and sequence similarity.

The C-C chemokines include potent chemoattractants of monocytes and lymphocytes such as monocyte chemoattractant proteins 1-3 (MCP-1, MCP-2 and MCP-3), RANTES (Regulated on activation, Normal T expressed and Secreted), eotaxin and the macrophage inflammatory proteins 1α and 1β (MIP-1α and MIP-1β).

The C-X-C chemokines include several potent chemoattractants and activators of neutrophils such as interleukin-8 (IL-8) and neutrophil-activating peptide 2 (NAP 2).

Studies have demonstrated that the actions of chemokines are mediated by subfamilies of G-protein coupled receptors, among which there are the receptors designated CCR1, CCR2, CCR3, CCR4, CCR5, CCR6, CCR7, CCR8, CCR9, CCR10, CXCR1, CXCR2, CXCR3, CXCR4, CXCR5 and CX3CR1. These receptors represent good targets for drug development since agents, which modulate these receptors would be useful in the treatment of disorders and diseases such as those mentioned above.

Published patent application WO-2006/067401 disclose piperazine urea compounds useful as modulators of the CCR2b receptor including 4-[4-tert-butylpiperazin-2-yl)carbonyl]-N-(3,4-dichlorophenyl)piperazine-1-carboxamide.

The present invention relates to a compound of formula (I) as a base or as a pharmaceutically acceptable salt thereof,

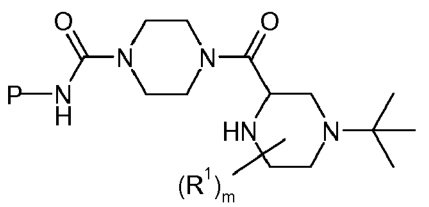

(I)

wherein P is a phenyl or a monocyclic or bicyclic heteroaryl having up to 9 ring atoms and comprising up to 3 heteroatoms independently selected from N, O, and S, wherein the phenyl or the heteroaryl is optionally substituted by up to 3 substituents independently selected from halogen, cyano, $CF_3$, $SC_{1-4}$ haloalkyl, $SC_{1-4}$ alkyl, $OC_{1-4}$ haloalkyl, $OC_{14}$alkyl, $OC_{1-4}$ alkylphenyl, $C_{1-4}$ alkyl, $C_{3-4}$ cycloalkyl, $C_{1-3}$ alkoxy, $C_{1-3}$ thioalkoxy, pentafluoroethyl, pentafluoroethoxy, phenyl, phen$C_{1-4}$alkyl, phen$C_{1-4}$alkoxy, phen$C_{14}$alkoxy$C_{1-4}$alkyl, heteroaryl, hetero$C_{1-4}$alkyl, hetero$C_{1-3}$alkoxy, and hetero$C_{1-3}$alkoxy$C_{14}$alkyl, wherein any substituent heteroaryl has up to 6 ring atoms and comprises up to 3 heteroatoms independently selected from N, O and S, wherein any substituent phenyl or heteroaryl ring is optionally substituted by up to 3 substituents independently selected from halogen, $C_{1-4}$ alkyl, $C_{3-4}$ cycloalkyl, $C_{1-3}$ alkoxy, $C_{1-3}$ thioalkoxy, pentafluoroethyl, pentafluoroethoxy, and cyano, wherein any alkyl or alkoxy is optionally substituted by up to three halogens; $R^1$ is $C_{1-4}$ alkyl; and m is 0, 1 or 2; and provided the formula I compound is not 4-[4-tert-butylpiperazin-2-yl)carbonyl]-N-(3,4-dichlorophenyl)piperazine-1-carboxamide.

Another embodiment relates to a compound according to formula (I) as a base or as a pharmaceutically acceptable salt thereof, wherein P is a phenyl or a monocyclic heteroaryl having up to 9 ring atoms and comprising up to 3 heteroatoms independently selected from N, O and S, wherein the phenyl or the heteroaryl is optionally substituted by up to 3 substituents independently selected from halogen, cyano, $CF_3$, $SC_{1-4}$ haloalkyl, $SC_{14}$ alkyl, $OC_{1-4}$ haloalkyl, $OC_{1-4}$alkyl, $OC_{1-4}$ alkylphenyl, $C_{1-4}$ alkyl, $C_{3-4}$ cycloalkyl, $C_{1-3}$ alkoxy, $C_{1-3}$ thioalkoxy, pentafluoroethyl, pentafluoroethoxy, phenyl, phen$C_{1-4}$alkyl, phen$C_{1-4}$alkoxy, phen$C_{1-4}$alkoxy$C_{1-4}$alkyl, heteroaryl, hetero$C_{1-4}$alkyl, hetero$C_{1-3}$alkoxy, and hetero$C_{1-3}$alkoxy$C_{1-4}$alkyl, wherein any substituent heteroaryl has up to 6 ring atoms and comprises up to 3 heteroatoms independently selected from N, O and S, and any substituent phenyl or heteroaryl ring is optionally substituted by up to 3 substituents independently selected from halogen, $C_{1-4}$ alkyl, $C_{3-4}$ cycloalkyl, $C_{1-3}$ alkoxy, $C_{1-3}$ thioalkoxy,

pentafluoroethyl, pentafluoroethoxy, and cyano; wherein any alkyl or alkoxy is optionally substituted by up to three halogens; $R^1$ is $C_{1-4}$ alkyl; and m is 0, 1 or 2.

A further embodiment relates to a compound according to formula (I) as a base or as a pharmaceutically acceptable salt thereof, wherein P is a phenyl or a monocyclic heteroaryl having up to 9 ring atoms and comprising up to 3 heteroatoms independently selected from N, O and S, wherein the phenyl or the heteroaryl is optionally substituted by up to 3 substituents independently selected from halogen, cyano, $CF_3$, $SC_{1-4}$ haloalkyl, $SC_{14}$ alkyl, $OC_{1-4}$ haloalkyl, $OC_{1-4}$alkyl, $OC_{1-4}$ alkylphenyl, $C_{1-4}$ alkyl, $C_{3-4}$ cycloalkyl, $C_{1-3}$ alkoxy, $C_{1-3}$ thioalkoxy, pentafluoroethyl, pentafluoroethoxy, phenyl, phen$C_{1-4}$alkyl, phen$C_{1-4}$alkoxy, phen$C_{1-4}$alkoxy$C_{1-4}$alkyl, heteroaryl, hetero$C_{1-4}$alkyl, hetero$C_{1-3}$alkoxy, and hetero$C_{1-3}$alkoxy$C_{1-4}$alkyl; $R^1$ is $C_{1-4}$ alkyl; and m is 0 or 1.

A still further embodiment relates to a compound according to formula (I) as a base or as a pharmaceutically acceptable salt thereof, wherein P is phenyl optionally substituted by up to 3 substituents independently selected from halogen, cyano, $CF_3$, $SC_{1-4}$ haloalkyl, $SC_{1-4}$ alkyl, $OC_{1-4}$ haloalkyl, $OC_{1-4}$alkyl, $OC_{1-4}$ alkylphenyl, $C_{1-4}$ alkyl, $C_{3-4}$ cycloalkyl, $C_{1-3}$ alkoxy, $C_{1-3}$ thioalkoxy, pentafluoroethyl, pentafluoroethoxy, phenyl, phen$C_{1-4}$alkyl, phen$C_{1-4}$alkoxy, and phen$C_{1-4}$alkoxy$C_{1-4}$alkyl.

A yet further embodiment relates to a compound according to formula (I) as a base or as a pharmaceutically acceptable salt thereof, wherein P is phenyl optionally substituted by up to 3 substituents independently selected from halogen, cyano, $CF_3$, $SC_{1-4}$ haloalkyl, $OC_{1-4}$ haloalkyl, $OC_{1-4}$ alkylphenyl, $C_{1-4}$ alkyl, $C_{3-4}$ cycloalkyl, $C_{1-3}$ alkoxy, $C_{1-3}$ thioalkoxy, and pentafluoroethyl.

A still yet further embodiment relates to a compound according to formula (I) as a base or as a pharmaceutically acceptable salt thereof, wherein P is phenyl substituted by up to 3 substituents independently selected from halogen, cyano, $CF_3$, $SC_{1-4}$ haloalkyl, $OC_{1-4}$ haloalkyl, $OC_{1-4}$ alkylphenyl, and $C_{1-4}$ alkyl.

According to another embodiment, P is phenyl.

In still another embodiment, said phenyl is substituted by 2 substituents selected from halogen.

In yet another embodiment, said halogens are selected from chloro and fluoro.

Another embodiment relates to a compound according to formula (I) as a base or as a pharmaceutically acceptable salt thereof, wherein P is a monocyclic heteroaryl having up to 6 ring atoms and comprising up to 3 heteroatoms independently selected from N, O and S, wherein said heteroaryl is optionally substituted by up to 3 substituents independently selected from halogen, cyano, $CF_3$, $SC_{1-4}$ haloalkyl, $SC_{1-4}$ alkyl, $OC_{1-4}$ haloalkyl, $OC_{14}$alkyl, $OC_{1-4}$ alkylphenyl, $C_{1-4}$ alkyl, $C_{3-4}$ cycloalkyl, $C_{1-3}$ alkoxy, $C_{1-3}$ thioalkoxy, pentafluoroethyl, pentafluoroethoxy, phenyl, phen$C_{1-4}$alkyl, phen$C_{1-4}$alkoxy, and phen$C_{14}$alkoxy$C_{1-4}$alkyl.

In a still yet further embodiment, said monocyclic heteroaryl is selected from furyl, thienyl, pyrrolyl, pyrrolidinyl, imidazolyl, thiazolyl, tetrazolyl, oxazolyl, isoxazolyl, pyrazolyl, pyridyl, pyrimidinyl, and pyrazinyl.

In an even still yet further embodiment, said monocyclic heteroaryl is substituted by up to 3 substituents independently selected from halogen, cyano, $CF_3$, $SC_{1-4}$ haloalkyl, $OC_{1-4}$ haloalkyl, $OC_{1-4}$ alkylphenyl, and $C_{1-4}$ alkyl.

Another embodiment relates to a compound according to formula (I) as a base or as a pharmaceutically acceptable salt thereof, wherein m is 0.

Yet another embodiment relates to a compound according to formula (I) as a base or as a pharmaceutically acceptable salt thereof, wherein P is a phenyl or a monocyclic heteroaryl having up to 5 ring atoms and comprising up to 3 heteroatoms independently selected from N, O and S, wherein the phenyl or the heteroaryl is optionally substituted by up to 3 substituents independently selected from halogen, cyano, $CF_3$, $SC_{1-4}$ haloalkyl, $SC_{14}$ alkyl, $OC_{1-4}$ haloalkyl, $OC_{1-4}$alkyl, $OC_{1-4}$ alkylphenyl, and $C_{1-4}$ alkyl; and m is 0.

The present invention also relates to a compound selected from 4-[(2R)-4-tert-butylpiperazine-2-carbonyl]-N[3(trifluoromethyl)phenyl] piperazine-1-carboxamide; 4-[(2R)-4-tert-butylpiperazine-2-carbonyl]-N-(3-chlorophenyl)piperazine-1-carboxamide; 4-[(2R)-4-tert-butylpiperazine-2-carbonyl]-N-[3-(trifluoromethylsulfanyl)phenyl] piperazine-1-carboxamide; N-(4-bromophenyl)-4-[(2R)-4-tert-butylpiperazine-2-carbonyl]piperazine-1-carboxamide; 4-[(2R)-4-tert-butylpiperazine-2-carbonyl]-N-[4-fluoro-3(trifluoromethyl) phenyl] piperazine-1-carboxamide; 4-[(2R)-4-tert-butylpiperazine-2-carbonyl]-N-[4-methyl-3-(trifluoromethyl) phenyl]piperazine-1-carboxamide; 4-[(2R)-4-tert-butylpiperazine-2-carbonyl]-N-(3-chloro-4-fluorophenyl) piperazine-1-carboxamide; 4-[(2R)-4-tert-butylpiperazine-2-carbonyl]-N-[4-cyano-3-(trifluoromethyl) phenyl]piperazine-1-carboxamide; 4-[(2R)-4-tert-butylpiperazine-2-carbonyl]-N-(4-chloro-3-fluorophenyl) piperazine-1-carboxamide; 4-[(2R)-4-tert-butylpiperazine-2-carbonyl]-N-[3-(1,1,2,2-tetrafluoroethoxy) phenyl]piperazine-1-carboxamide; 4-[(2R)-4-tert-butylpiperazine-2-carbonyl]-N-(4-chloro-3-methylphenyl) piperazine-1-carboxamide; 4-[(2R)-4-tert-butylpiperazine-2-carbonyl]-N-(3-phenylmethoxyphenyl) piperazine-1-carboxamide; 4-[(2R)-4-tert-butylpiperazine-2-carbonyl]-N-[3-chloro-4-(trifluoromethoxy) phenyl]piperazine-1-carboxamide; N-(5-bromo-4-methyl-1,3-thiazol-2-yl)-4-[(2R)-4-tert-butylpiperazine-2-carbonyl]piperazine-1-carboxamide; 4-[(2R)-4-tert-butylpiperazine-2-carbonyl]-N-(5-chloro-4-ethyl-1,3-thiazol-2-yl)piperazine-1-carboxamide; 4-[(2R)-4-tert-butylpiperazine-2-carbonyl]-N-(5-chloro-4-methyl-1,3-thiazol-2-yl)piperazine-1-carboxamide; and 4-[(2R)-4-tert-butylpiperazine-2-carbonyl]-N-[5-chloro-4-(trifluoromethyl)-1,3-thiazol-2-yl]piperazine-1-carboxamide; and each as a base or a pharmaceutically acceptable salt thereof.

Compounds of formula (I) as a base or a pharmaceutically acceptable salt thereof can be used in the treatment of diseases in which the chemokine receptor belongs to the C-C receptor subfamily. In, for example, one scenario the target chemokine receptor is the CCR2 receptor.

CCR2 is a receptor for the Monocyte chemoattractant protein-1 (MCP-1). MCP-1 is a member of the chemokine family of pro-inflammatory proteins that mediate leukocyte chemotaxis and activation. MCP-1 is a C-C chemokine that is a potent T-cell and monocyte chemoattractant. MCP-1 has been implicated in the pathophysiology of a large number of inflammatory diseases including, for example, rheumatoid arthritis, chronic obstructive pulmonary disease, atherosclerosis, inflammatory bowel disease, and neuropathic pain.

MCP-1 acts through the CCR2 receptor. MCP-2, MCP-3 and MCP-4 may also act, at least in part, through this receptor. Therefore in this specification, when reference is made to "inhibition or antagonism of MCP-1" or "MCP-1 mediated effects" this includes inhibition or antagonism of MCP-2 and/or MCP-3 and/or MCP-4 mediated effects when MCP-2 and/or MCP-3 and/or MCP-4 are acting through the CCR2 receptor.

Mechanistically CCR2 is involved in neuropathic pain, sensory nerve injury has been shown to increase both MCP-1 expression in dorsal root ganglia (DRG) sensory neurones [Tanaka *et al,* 2004, Zhang et al, 2006, W02004/110376 A2, White *et al,* 2005b] and CCR2 expression in both neuronal and non-neuronal DRG cells [White et al, 2005b, WO2004/110376, Abbadie *et al,* 2003]. This results in two mechanisms by which CCR2 may modulate pain. One is a direct mechanism, where up-regulation of CCR2 on sensory neurones increases neuronal excitability; neurones inappropriately depolarise and can spontaneously fire in response to tissue or neuronal MCP-1 [Oh *et al,* 2001, Qin *et al,* 2005, White *et al,* 2005, Sun *et al,* 2006]. The other mechanism is one in which elevated MCP-1 production from injured neurones recruits and/or activates CCR2+ immune cells. In turn, these CCR2+ immune cells in turn secrete pro-algesic substances that modulate pain processing. This indirect mechanism occurs both peripherally, where the CCR2+ cells are monocyte/macrophages, and centrally where the CCR2+ cells are microglial cells [Abbadie *et al,* 2003, Zhang et al 2007].

The term "$C_{m-n}$" or "$C_{m-n}$ group" refers to any group having m to n carbon atoms.

As used herein, "alkyl", used alone or as a suffix or prefix, is intended to include both branched and straight chain saturated aliphatic hydrocarbon groups having from 1 to 12 carbon atoms or if a specified number of carbon atoms is provided then that specific number would be intended. For example "$C_{1-6}$ alkyl" denotes alkyl having 1, 2, 3, 4, 5 or 6 carbon atoms. Examples of alkyl include, but are not limited to, methyl, ethyl, n-propyl, i-propyl, n-butyl, i-butyl, sec-butyl, t-butyl, pentyl, and hexyl. In the case where a subscript is the integer 0 (zero) the group to which the subscript refers to indicates that the group may be absent, i.e. there is a direct bond between the groups.

The term "alkoxy", unless stated otherwise, refers to radicals of the general formula -O-R, wherein R is selected from a hydrocarbon radical. For example "$C_{1-6}$ alkoxy" denotes alkoxy having 1, 2, 3, 4, 5 or 6 carbon atoms. Examples of alkoxy include, but are not limited to, methoxy, ethoxy, propoxy, isopropoxy, butoxy, t-butoxy and isobutoxy.

As used herein, "alkenyl", used alone or as a suffix or prefix, is intended to include both branched and straight chain aliphatic hydrocarbon groups comprising at least one carbon-carbon double bond (-C=C-) and having from 2 to 12 carbon atoms or if a specified number of carbon atoms is provided then that specific number would be intended. For example "$C_{2-6}$ alkenyl" denotes alkenyl having 2, 3, 4, 5 or 6 carbon atoms.

As used herein, "alkynyl", ", used alone or as a suffix or prefix, is intended to include both branched and straight chain aliphatic hydrocarbon groups comprising at least one carbon-carbon triple bond (-C≡C-) and having from 2 to 12 carbon atoms or if a specified number of carbon atoms is provided then that specific number would be intended. For example "$C_{2-6}$ alkynyl" denotes alkynyl having 2, 3, 4, 5 or 6 carbon atoms.

The term "halo" includes fluoro, chloro, bromo and iodo. References to aryl groups include aromatic carbocylic groups such as phenyl and naphthyl.

The term "heterocyclyl" includes aromatic or non-aromatic rings, or partially unsaturated ring systems, for example containing from 5-7 ring atoms, at least one of which is a heteroatom such as oxygen, sulphur or nitrogen. Rings may be mono- or bicyclic. They may also contain bridges, in particular alkyl bridges. Examples of such groups include furyl, thienyl, pyrrolyl, pyrrolidinyl, imidazolyl, thiazolyl, tetrazolyl, oxazolyl, isoxazolyl, pyrazolyl, pyridyl, pyrimidinyl, pyrazinyl, pyridazinyl, triazinyl, quinolinyl, iosquinolinyl, quinoxalinyl, benzthiazolyl, benzoxazolyl, benzothienyl, benzofuranyl, tetrahydrofuryl, chromanyl, piperidinyl, 1,2,3,4-tetrahydroquinolinyl, 1,2,3,4-tetrahydroisoquinolinyl, piperazinyl, quinoxalinyl, quinazolinyl, cinnolinyl, indolyl, indolinyl, benzimidazolyl, indazolyl, oxazolyl, benzoxazolyl, isoxazolyl, morpholinyl, dioxolane, benzodioxolane, 4H-1,4-benzoxazinyl, 4H-1,4-benzothiazinyl, 1,2,3-triazolyl, 1,2,4-triazolyl, oxadiazolyl, furazanyl, thiadiazolyl, dibenzofuranyl, dibenzothienyl oxiranyl, oxetanyl, azetidinyl, oxepanyl, oxazepanyl, tetrahydro-1,4-thiazinyl, 1,1-dioxotetrahydro-1,4-thiazinyl, homopiperidinyl, homopiperazinyl, dihydropyridinyl, tetrahydropyridinyl, dihydropyrimidinyl, tetrahydropyrimidinyl, tetrahydrothienyl, tetrahydrothiopyranyl or thiomorpholinyl.

"Heteroaryl" refers to those heterocyclyl groups described above which has an aromatic character. The term "aralkyl" refers to aryl substituted alkyl groups such as benzyl. The terms "aryl" and "heteroaryl" include non-condensed ring systems such as biphenyl.

As used herein, "pharmaceutically acceptable salts" refer to derivatives of the disclosed compounds wherein the parent compound is modified by making acid or base salts thereof. Examples of pharmaceutically acceptable salts include, but are not limited to, mineral or organic acid salts of basic residues such as amines; alkali or organic salts of acidic residues such as carboxylic acids; and the like. The pharmaceutically acceptable salts include the conventional non-toxic salts or the quaternary ammonium salts of the parent compound formed, for example, from non-toxic inorganic or organic acids. For example, such conventional non-toxic salts include those derived from inorganic acids such as hydrochloric acid.

The pharmaceutically acceptable salts of the present invention can be synthesized from the parent compound that contains a basic or acidic moiety by conventional chemical methods. Generally, such salts can be prepared by reacting the free acid or base forms of these compounds with a stoichiometric amount of the appropriate base or acid in water or in an organic solvent, or in a mixture of the two; generally, nonaqueous media like diethyl ether, ethyl acetate, ethanol, isopropanol, or acetonitrile are used.

Compounds of the invention show unexpected properties when compared to compounds disclosed in for example WO-2006/067401. For example, a compound of the invention may have increased potency and/or increased margins vs hERG.

The compounds of Formula I and pharmaceutically acceptable salts thereof may be prepared as follows:

(a) by the reaction of a compound of Formula (II),

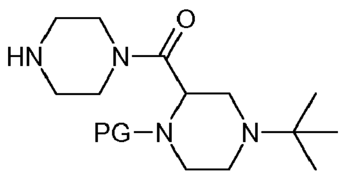

(II)

wherein PG is a protecting group, for example, benzyloxycarbonyl, benzyl or *tert*-butyloxycarbonyl with an isocyanate of Formula (III)

P-N=C=O (III)

or, with a carbamate of Formula (IV).

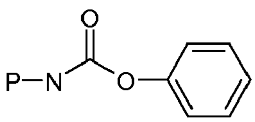

(IV)

wherein P is defined above, followed by removal of the protecting group or

(b) by the amide coupling of a compound of Formula (V),

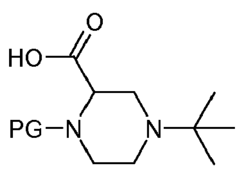

(V)

wherein PG is a protecting group, with a compound of Formula (VI)

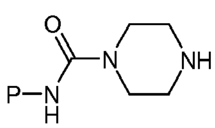

(VI)

wherein P is defined above, followed by removal of the protecting group, and, if required, by conversion to a pharmaceutically acceptable salt thereof.

When the protecting group is benzyloxycarbonyl or benzyl then removal can be effected by hydrogenation (for example hydrogen in the presence of palladium on carbon catalyst). When the protecting group is tert-butyloxycarbonyl removal may be effected by treatment with acid (such as hydrochloric acid or trifluoroacetic acid).

Convenient protecting groups and details of processes for adding and removing such groups may be found in "Protective Groups in Organic Synthesis", 3rd Edition (1999) by Greene and Wuts.

The compounds of Formula I may be prepared as follows:

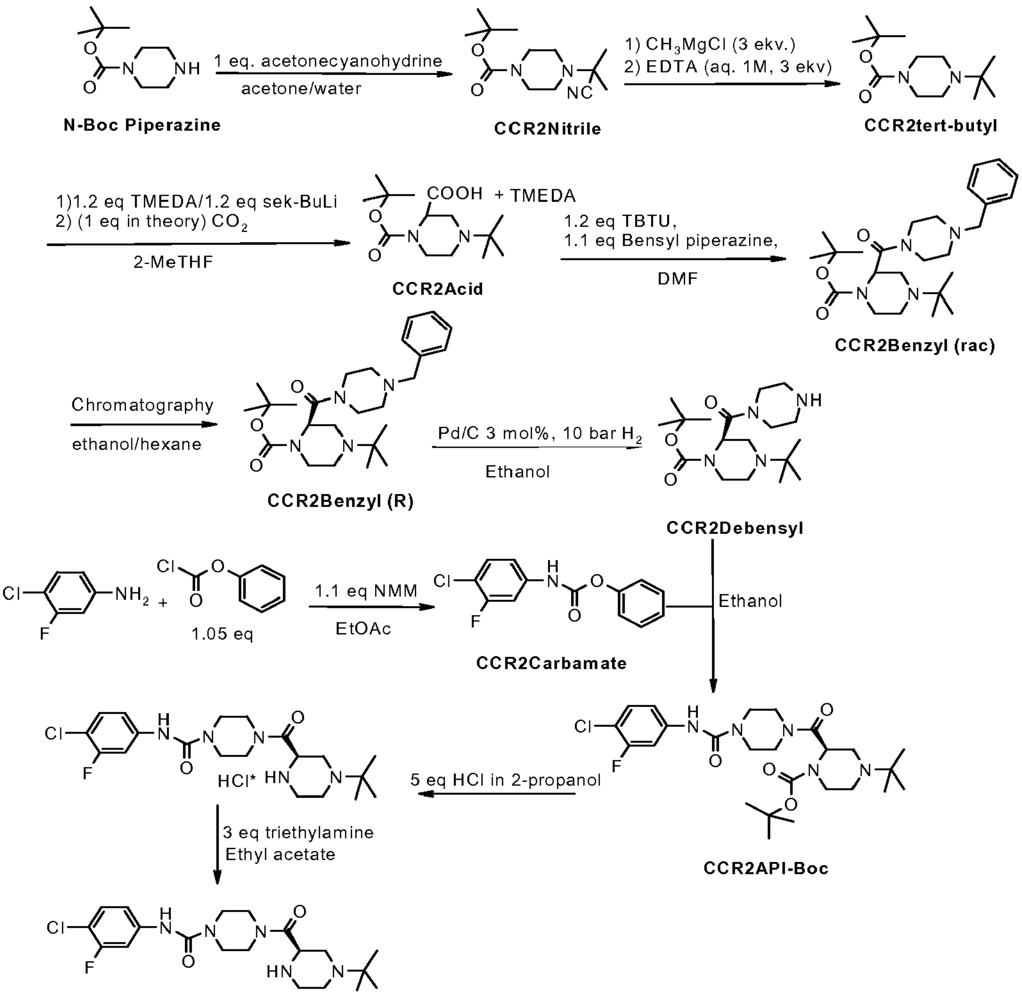


A compound of formula (I), as a base or a pharmaceutically acceptable salt thereof, may be used in the treatment of:

1. Respiratory tract: obstructive diseases of the airways including: asthma, including bronchial, allergic, intrinsic, extrinsic, exercise-induced, drug-induced (including aspirin and NSAID-induced) and dust-induced asthma, both intermittent and persistent and of all severities, and other causes of airway hyper-responsiveness; chronic obstructive pulmonary disease (COPD); bronchitis, including infectious and eosinophilic bronchitis; emphysema; bronchiectasis; cystic fibrosis; sarcoidosis; farmer's lung and related diseases; hypersensitivity pneumonitis; lung fibrosis, including cryptogenic fibrosing alveolitis, idiopathic interstitial pneumonias, fibrosis complicating anti-neoplastic therapy and chronic infection, including tuberculosis and aspergillosis and other fungal infections; complications of lung transplantation; vasculitic and thrombotic disorders of the lung vasculature, and pulmonary hypertension; antitussive activity including treatment of chronic cough associated with inflammatory and secretory conditions of the airways, and iatrogenic cough; acute and chronic rhinitis including rhinitis medicamentosa, and vasomotor rhinitis; perennial and seasonal allergic rhinitis including rhinitis nervosa (hay fever); nasal polyposis; acute viral infection including the common cold, and infection due to respiratory syncytial virus, influenza, coronavirus (including SARS) and adenovirus;
2. Bone and joints: arthritides associated with or including osteoarthritis/osteoarthrosis, both primary and secondary to, for example, congenital hip dysplasia; cervical and lumbar spondylitis, and low back and neck pain; rheumatoid

arthritis and Still's disease; seronegative spondyloarthropathies including ankylosing spondylitis, psoriatic arthritis, reactive arthritis and undifferentiated spondarthropathy; septic arthritis and other infection-related arthopathies and bone disorders such as tuberculosis, including Potts' disease and Poncet's syndrome; acute and chronic crystal-induced synovitis including urate gout, calcium pyrophosphate deposition disease, and calcium apatite related tendon, bursal and synovial inflammation; Behcet's disease; primary and secondary Sjogren's syndrome; systemic sclerosis and limited scleroderma; systemic lupus erythematosus, mixed connective tissue disease, and undifferentiated connective tissue disease; inflammatory myopathies including dermatomyositits and polymyositis; polymalgia rheumatica; juvenile arthritis including idiopathic inflammatory arthritides of whatever joint distribution and associated syndromes, and rheumatic fever and its systemic complications; vasculitides including giant cell arteritis, Takayasu's arteritis, Churg-Strauss syndrome, polyarteritis nodosa, microscopic polyarteritis, and vasculitides associated with viral infection, hypersensitivity reactions, cryoglobulins, and paraproteins; low back pain; Familial Mediterranean fever, Muckle-Wells syndrome, and Familial Hibernian Fever, Kikuchi disease; drug-induced arthalgias, tendonititides, and myopathies;

3. Pain and connective tissue remodelling of musculoskeletal disorders due to injury [for example sports injury] or disease: arthitides (for example rheumatoid arthritis, osteoarthritis, gout or crystal arthropathy), other joint disease (such as intervertebral disc degeneration or temporomandibular joint degeneration), bone remodelling disease (such as osteoporosis, Paget's disease or osteonecrosis), polychondritits, scleroderma, mixed connective tissue disorder, spondyloarthropathies or periodontal disease (such as periodontitis);

4. Skin: psoriasis, atopic dermatitis, contact dermatitis or other eczematous dermatoses, and delayed-type hypersensitivity reactions; phyto- and photodermatitis; seborrhoeic dermatitis, dermatitis herpetiformis, lichen planus, lichen sclerosus et atrophica, pyoderma gangrenosum, skin sarcoid, discoid lupus erythematosus, pemphigus, pemphigoid, epidermolysis bullosa, urticaria, angioedema, vasculitides, toxic erythemas, cutaneous eosinophilias, alopecia areata, male-pattern baldness, Sweet's syndrome, Weber-Christian syndrome, erythema multiforme; cellulitis, both infective and non-infective; panniculitis; cutaneous lymphomas, non-melanoma skin cancer and other dysplastic lesions; drug-induced disorders including fixed drug eruptions;

5. Eyes: blepharitis; conjunctivitis, including perennial and vernal allergic conjunctivitis; iritis; anterior and posterior uveitis; choroiditis; autoimmune; degenerative or inflammatory disorders affecting the retina; ophthalmitis including sympathetic ophthalmitis; sarcoidosis; infections including viral, fungal, and bacterial;

6. Gastrointestinal tract: glossitis, gingivitis, periodontitis; oesophagitis, including reflux; eosinophilic gastro-enteritis, mastocytosis, Crohn's disease, colitis including ulcerative colitis, proctitis, pruritis ani; coeliac disease, irritable bowel syndrome, and food-related allergies which may have effects remote from the gut (for example migraine, rhinitis or eczema);

7. Abdominal: hepatitis, including autoimmune, alcoholic and viral; fibrosis and cirrhosis of the liver; cholecystitis; pancreatitis, both acute and chronic;

8. Genitourinary: nephritis including interstitial and glomerulonephritis; nephrotic syndrome; cystitis including acute and chronic (interstitial) cystitis and Hunner's ulcer; acute and chronic urethritis, prostatitis, epididymitis, oophoritis and salpingitis; vulvo-vaginitis; Peyronie's disease; erectile dysfunction (both male and female);

9. Allograft rejection: acute and chronic following, for example, transplantation of kidney, heart, liver, lung, bone marrow, skin or cornea or following blood transfusion; or chronic graft versus host disease;

10. CNS: Alzheimer's disease and other dementing disorders including CJD and nvCJD; amyloidosis; multiple sclerosis and other demyelinating syndromes; cerebral atherosclerosis and vasculitis; temporal arteritis; myasthenia gravis; acute and chronic pain (acute, intermittent or persistent, whether of central or peripheral origin) including visceral pain, headache, migraine, trigeminal neuralgia, atypical facial pain, joint and bone pain, pain arising from cancer and tumor invasion, neuropathic pain syndromes including diabetic, post-herpetic, and HIV-associated neuropathies; neurosarcoidosis; central and peripheral nervous system complications of malignant, infectious or autoimmune processes;

11. Other auto-immune and allergic disorders including Hashimoto's thyroiditis, Graves' disease, Addison's disease, diabetes mellitus, idiopathic thrombocytopaenic purpura, eosinophilic fasciitis, hyper-IgE syndrome, antiphospholipid syndrome;

12. Other disorders with an inflammatory or immunological component; including HIV infection and acquired immune deficiency syndrome (AIDS), leprosy, Sezary syndrome, and paraneoplastic syndromes;

13. Cardiovascular: atherosclerosis, affecting the coronary and peripheral circulation; pericarditis; myocarditis, inflammatory and auto-immune cardiomyopathies including myocardial sarcoid; ischaemic reperfusion injuries; endocarditis, valvulitis, and aortitis including infective (for example syphilitic); vasculitides; disorders of the proximal and peripheral veins including phlebitis and thrombosis, including deep vein thrombosis and complications of varicose veins;

14. Oncology: treatment of common cancers including prostate, breast, lung, ovarian, pancreatic, bowel and colon, stomach, skin and brain tumors and malignancies affecting the bone marrow (including the leukaemias) and lymphoproliferative systems, such as Hodgkin's and non-Hodgkin's lymphoma; including the prevention and treatment of metastatic disease and tumour recurrences, and paraneoplastic syndromes; and

15. Gastrointestinal tract: Coeliac disease, proctitis, eosinopilic gastro-enteritis, mastocytosis, Crohn's disease, ulcerative colitis, microscopic colitis, indeterminant colitis, irritable bowel disorder, irritable bowel syndrome, non-inflammatory diarrhea, food-related allergies which have effects remote from the gut, e.g., migraine, rhinitis and eczema.

The invention further provides a compound of Formula (I) as defined above for use in the treatment of C-C-chemokine mediated disease such as inflammatory disease. When used in this way, the compounds are suitably formulated into pharmaceutical compositions, which further contain a pharmaceutically acceptable carrier and these form a further aspect of the invention. The compound is conveniently used for the treatment of CCR2b mediated inflammatory diseases and/or neuropathic pain.

The compounds of the present invention may be used in the treatment of neuropathic pain associated with a C-C chemokine mediated disease, condition or disorder such as those listed hereinbefore. In particular compounds of the invention may be used in the treatment of neuropathic pain associated with a CCR2b chemokine mediated disease, condition or disorder. The compounds of the present invention may also be use in the treatment of acute pain, chronic pain, back pain, cancer pain, and visceral pain.

Convenient examples include neuropathic pain associated with inflammatory diseases such as rheumatoid arthritis, osteoarthritis, asthma, allergic rhinitis, chronic obstructive pulmonary disease (COPD), psoriasis and inflammatory bowel disease.

In particular compounds of the invention may be used to treat neuropathic pain associated with arthritis and in particular osteoarthritis.

Furthermore, the invention provides the use of a compound of Formula (I) as defined above in the preparation of a medicament for treating C-C chemokine mediated disease, and in particular for the treatment of CCR2b mediated inflammatory diseases.

The invention further relates to combination therapies wherein a compound of the invention, or a pharmaceutically acceptable salt thereof, or a pharmaceutical composition or formulation comprising a compound of the invention, is administered concurrently or sequentially or as a combined preparation with another therapeutic agent or agents, for the treatment of one or more of the conditions listed.

In particular, for the treatment of the inflammatory diseases such as (but not restricted to) rheumatoid arthritis, osteoarthritis, asthma, allergic rhinitis, chronic obstructive pulmonary disease (COPD), psoriasis, neuropathic pain and inflammatory bowel disease, the compounds of the invention may be combined with agents listed below.

Non-steroidal anti-inflammatory agents (hereinafter NSAIDs) including non-selective cyclo-oxygenase COX-1 / COX-2 inhibitors whether applied topically or systemically (such as piroxicam, diclofenac, propionic acids such as naproxen, flurbiprofen, fenoprofen, ketoprofen and ibuprofen, fenamates such as mefenamic acid, indomethacin, sulindac, azapropazone, pyrazolones such as phenylbutazone, salicylates such as aspirin); selective COX-2 inhibitors (such as meloxicam, celecoxib, rofecoxib, valdecoxib, lumarocoxib, parecoxib and etoricoxib); cyclo-oxygenase inhibiting nitric oxide donors (CINODs); glucocorticosteroids (whether administered by topical, oral, intramuscular, intravenous, or intra-articular routes); methotrexate; leflunomide; hydroxychloroquine; d-penicillamine; auranofin or other parenteral or oral gold preparations; analgesics; diacerein; intra-articular therapies such as hyaluronic acid derivatives; and nutritional supplements such as glucosamine.

The present invention still further relates to the combination of a compound of the invention, or a pharmaceutically acceptable salt thereof, together with a cytokine or agonist or antagonist of cytokine function, (including agents which act on cytokine signalling pathways such as modulators of the SOCS system) including alpha-, beta-, and gamma-interferons; insulin-like growth factor type I (IGF-1); interleukins (IL) including IL1 to 17, and interleukin antagonists or inhibitors such as anakinra; tumour necrosis factor alpha (TNF-$\alpha$) inhibitors such as anti-TNF monoclonal antibodies (for example infliximab; adalimumab, and CDP-870) and TNF receptor antagonists including immunoglobulin molecules (such as etanercept) and low-molecular-weight agents such as pentoxyfylline.

In addition the invention relates to a combination of a compound of the invention, or a pharmaceutically acceptable salt thereof, with a monoclonal antibody targeting B-Lymphocytes (such as CD20 (rituximab), MRA-aIL16R and T-Lymphocytes, CTLA4-Ig, HuMax I1-15).

The present invention still further relates to the combination of a compound of the invention, or a pharmaceutically acceptable salt thereof, with a modulator of chemokine receptor function such as an antagonist of CCR1, CCR3, CCR4, CCR5, CCR6, CCR7, CCR8, CCR9, CCR10 and CCR11 (for the C-C family); CXCR1, CXCR2, CXCR3, CXCR4 and CXCR5 (for the C-X-C family) and $CX_3CR1$ for the C-$X_3$-C family.

The present invention further relates to the combination of a compound of the invention, or a pharmaceutically acceptable salt thereof, with an inhibitor of matrix metalloprotease (MMPs), i.e., the stromelysins, the collagenases, and the gelatinases, as well as aggrecanase; especially collagenase-1 (MMP-1), collagenase-2 (MMP-8), collagenase-3

(MMP-13), stromelysin-1 (MMP-3), stromelysin-2 (MMP-10), and stromelysin-3 (MMP-11) and MMP-9 and MMP-12, including agents such as doxycycline.

The present invention still further relates to the combination of a compound of the invention, or a pharmaceutically acceptable salt thereof, and a leukotriene biosynthesis inhibitor, 5-lipoxygenase (5-LO) inhibitor or 5-lipoxygenase activating protein (FLAP) antagonist such as; zileuton; ABT-761; fenleuton; tepoxalin; Abbott-79175; Abbott-85761; a N-(5-substituted)-thiophene-2-alkylsulfonamide; 2,6-di-tert-butylphenolhydrazones; a methoxytetrahydropyrans such as Zeneca ZD-2138; the compound SB-210661; a pyridinyl-substituted 2-cyanonaphthalene compound such as L-739,010; a 2-cyanoquinoline compound such as L-746,530; or an indole or quinoline compound such as MK-591, MK-886, and BAY x 1005.

The present invention further relates to the combination of a compound of the invention, or a pharmaceutically acceptable salt thereof, and a receptor antagonist for leukotrienes (LT) B4, LTC4, LTD4, and LTE4. Selected from the group consisting of the phenothiazin-3-1s such as L-651,392; amidino compounds such as CGS-25019c; benzoxalamines such as ontazolast; benzenecarboximidamides such as BIIL 284/260; and compounds such as zafirlukast, ablukast, montelukast, pranlukast, verlukast (MK-679), RG-12525, Ro-245913, iralukast (CGP 45715A), and BAY x 7195.

The present invention still further relates to the combination of a compound of the invention, or a pharmaceutically acceptable salt thereof, and a phosphodiesterase (PDE) inhibitor such as a methylxanthanine including theophylline and aminophylline; a selective PDE isoenzyme inhibitor including a PDE4 inhibitor an inhibitor of the isoform PDE4D, or an inhibitor of PDE5.

The present invention further relates to the combination of a compound of the invention, or a pharmaceutically acceptable salt thereof, and a histamine type 1 receptor antagonist such as cetirizine, loratadine, desloratadine, fexofenadine, acrivastine, terfenadine, astemizole, azelastine, levocabastine, chlorpheniramine, promethazine, cyclizine, or mizolastine; applied orally, topically or parenterally.

The present invention still further relates to the combination of a compound of the invention, or a pharmaceutically acceptable salt thereof, and a proton pump inhibitor (such as omeprazole) or a gastroprotective histamine type 2 receptor antagonist.

The present invention further relates to the combination of a compound of the invention, or a pharmaceutically acceptable salt thereof, and an antagonist of the histamine type 4 receptor.

The present invention still further relates to the combination of a compound of the invention, or a pharmaceutically acceptable salt thereof, and an alpha-1/alpha-2 adrenoceptor agonist vasoconstrictor sympathomimetic agent, such as propylhexedrine, phenylephrine, phenylpropanolamine, ephedrine, pseudoephedrine, naphazoline hydrochloride, oxymetazoline hydrochloride, tetrahydrozoline hydrochloride, xylometazoline hydrochloride, tramazoline hydrochloride or ethylnorepinephrine hydrochloride.

The present invention further relates to the combination of a compound of the invention, or a pharmaceutically acceptable salt thereof, and an anticholinergic agents including muscarinic receptor (M1, M2, and M3) antagonist such as atropine, hyoscine, glycopyrrrolate, ipratropium bromide, tiotropium bromide, oxitropium bromide, pirenzepine or telenzepine.

The present invention still further relates to the combination of a compound of the invention, or a pharmaceutically acceptable salt thereof, and a beta-adrenoceptor agonist (including beta receptor subtypes 1-4) such as isoprenaline, salbutamol, formoterol, salmeterol, terbutaline, orciprenaline, bitolterol mesylate, or pirbuterol, or a chiral enantiomer thereof.

The present invention further relates to the combination of a compound of the invention, or a pharmaceutically acceptable salt thereof, and a chromone, such as sodium cromoglycate or nedocromil sodium.

The present invention still further relates to the combination of a compound of the invention, or a pharmaceutically acceptable salt thereof, with a glucocorticoid, such as flunisolide, triamcinolone acetonide, beclomethasone dipropionate, budesonide, fluticasone propionate, ciclesonide or mometasone furoate.

The present invention further relates to the combination of a compound of the invention, or a pharmaceutically acceptable salt thereof, with an agent that modulates a nuclear hormone receptor such as PPARs, for example rosiglitazone.

The present invention further relates to the combination of a compound of the invention, or a pharmaceutically acceptable salt thereof with gabapentin, lidoderm, pregablin and equivalents and pharmaceutically active isomer(s) and metabolite(s) thereof.

The present invention further relates to the combination of a compound of the invention, or a pharmaceutically acceptable salt thereof with celecoxib, etoricoxib, lumiracoxib, rofecoxib, valdecoxib, diclofenac, loxoprofen, naproxen, paracetamol and equivalents and pharmaceutically active isomer(s) and metabolite(s) thereof.

The present invention still further relates to the combination of a compound of the invention, or a pharmaceutically acceptable salt thereof, together with an immunoglobulin (Ig) or Ig preparation or an antagonist or antibody modulating Ig function such as anti-IgE (for example omalizumab).

The present invention further relates to the combination of a compound of the invention, or a pharmaceutically

acceptable salt thereof, and another systemic or topically-applied anti-inflammatory agent, such as thalidomide or a derivative thereof, a retinoid, dithranol or calcipotriol.

The present invention still further relates to the combination of a compound of the invention, or a pharmaceutically acceptable salt thereof, and combinations of aminosalicylates and sulfapyridine such as sulfasalazine, mesalazine, balsalazide, and olsalazine; and immunomodulatory agents such as the thiopurines, and corticosteroids such as budesonide.

The present invention further relates to the combination of a compound of the invention, or a pharmaceutically acceptable salt thereof, together with an antibacterial agent such as a penicillin derivative, a tetracycline, a macrolide, a beta-lactam, a fluoroquinolone, metronidazole, an inhaled aminoglycoside; an antiviral agent including acyclovir, famciclovir, valaciclovir, ganciclovir, cidofovir, amantadine, rimantadine, ribavirin, zanamavir and oseltamavir; a protease inhibitor such as indinavir, nelfinavir, ritonavir, and saquinavir; a nucleoside reverse transcriptase inhibitor such as didanosine, lamivudine, stavudine, zalcitabine or zidovudine; or a non-nucleoside reverse transcriptase inhibitor such as nevirapine or efavirenz.

The present invention still further relates to the combination of a compound of the invention, or a pharmaceutically acceptable salt thereof, and a cardiovascular agent such as a calcium channel blocker, a beta-adrenoceptor blocker, an angiotensin-converting enzyme (ACE) inhibitor, an angiotensin-2 receptor antagonist; a lipid lowering agent such as a statin or a fibrate; a modulator of blood cell morphology such as pentoxyfylline; thrombolytic, or an anticoagulant such as a platelet aggregation inhibitor.

The present invention further relates to the combination of a compound of the invention, or a pharmaceutically acceptable salt thereof, and a CNS agent such as an antidepressant (such as sertraline), an anti-Parkinsonian drug (such as deprenyl, L-dopa, ropinirole, pramipexole, a MAOB inhibitor such as selegine and rasagiline, a comP inhibitor such as tasmar, an A-2 inhibitor, a dopamine reuptake inhibitor, an NMDA antagonist, a nicotine agonist, a dopamine agonist or an inhibitor of neuronal nitric oxide synthase), or an anti-Alzheimer's drug such as donepezil, rivastigmine, tacrine, a COX-2 inhibitor, propentofylline or metrifonate.

The present invention still further relates to the combination of a compound of the invention, or a pharmaceutically acceptable salt thereof, and an agent for the treatment of acute or chronic pain, such as a centrally or peripherally-acting analgesic (for example an opioid or derivative thereof), carbamazepine, gabapentin, pregabalin, phenytoin, sodium valproate, amitryptiline or other anti-depressant agent-s, paracetamol, CB1 agonist, muscarinic agonist, TRPV-1 antagonist, mGluR5 agonist or a non-steroidal anti-inflammatory agent.

The present invention further relates to the combination of a compound of the invention, or a pharmaceutically acceptable salt thereof, together with a parenterally or topically-applied (including inhaled) local anaesthetic agent such as lignocaine or a derivative thereof.

A compound of the present invention, or a pharmaceutically acceptable salt thereof, can also be used in combination with an anti-osteoporosis agent including a hormonal agent such as raloxifene, or a biphosphonate such as alendronate.

The present invention still further relates to the combination of a compound of the invention, or a pharmaceutically acceptable salt thereof, together with a: (i) tryptase inhibitor; (ii) platelet activating factor (PAF) antagonist; (iii) interleukin converting enzyme (ICE) inhibitor; (iv) IMPDH inhibitor; (v) adhesion molecule inhibitors including VLA-4 antagonist; (vi) cathepsin; (vii) kinase inhibitor such as an inhibitor of tyrosine kinase (such as Btk, Itk, Jak3 or MAP, for example Gefitinib or Imatinib mesylate), a serine / threonine kinase (such as an inhibitor of a MAP kinase such as p38, JNK, protein kinase A, B or C, or IKK), or a kinase involved in cell cycle regulation (such as a cylin dependent kinase); (viii) glucose-6 phosphate dehydrogenase inhibitor; (ix) kinin-B.sub1. - or B.sub2. -receptor antagonist; (x) anti-gout agent, for example colchicine; (xi) xanthine oxidase inhibitor, for example allopurinol; (xii) uricosuric agent, for example probenecid, sulfinpyrazone or benzbromarone; (xiii) growth hormone secretagogue; (xiv) transforming growth factor (TGF$\beta$); (xv) platelet-derived growth factor (PDGF); (xvi) fibroblast growth factor for example basic fibroblast growth factor (bFGF); (xvii) granulocyte macrophage colony stimulating factor (GM-CSF); (xviii) capsaicin cream; (xix) tachykinin NK.sub1 or NK.sub3. receptor antagonist such as NKP-608C, SB-233412 (talnetant) or D-4418; (xx) elastase inhibitor such as UT-77 or ZD-0892; (xxi) TNF-alpha converting enzyme inhibitor (TACE); (xxii) induced nitric oxide synthase (iNOS) inhibitor; (xxiii) chemoattractant receptor-homologous molecule expressed on TH2 cells, (such as a CRTH2 antagonist); (xxiv) inhibitor of P38; (xxv) agent modulating the function of Toll-like receptors (TLR), (xxvi) agent modulating the activity of purinergic receptors such as P2X7 or P2X3; or (xxvii) inhibitor of transcription factor activation such as NFkB, API, or STATS.

A compound of the invention, or a pharmaceutically acceptable salt thereof, can also be used in combination with an existing therapeutic agent for the treatment of cancer, for example suitable agents include:

(i) an antiproliferative/antineoplastic drug or a combination thereof, as used in medical oncology, such as an alkylating agent (for example cis-platin, carboplatin, cyclophosphamide, nitrogen mustard, melphalan, chlorambucil, busulphan or a nitrosourea); an antimetabolite (for example an antifolate such as a fluoropyrimidine like 5-fluorouracil or tegafur,

raltitrexed, methotrexate, cytosine arabinoside, hydroxyurea, gemcitabine or paclitaxel); an antitumour antibiotic (for example an anthracycline such as adriamycin, bleomycin, doxorubicin, daunomycin, epirubicin, idarubicin, mitomycin-C, dactinomycin or mithramycin); an antimitotic agent (for example a vinca alkaloid such as vincristine, vinblastine, vindesine or vinorelbine, or a taxoid such as taxol or taxotere); or a topoisomerase inhibitor (for example an epipodophyllotoxin such as etoposide, teniposide, amsacrine, topotecan or a camptothecin);

(ii) a cytostatic agent such as an antioestrogen (for example tamoxifen, toremifene, raloxifene, droloxifene or iodoxyfene), an oestrogen receptor down regulator (for example fulvestrant), an antiandrogen (for example bicalutamide, flutamide, nilutamide or cyproterone acetate), a LHRH antagonist or LHRH agonist (for example goserelin, leuprorelin or buserelin), a progestogen (for example megestrol acetate), an aromatase inhibitor (for example as anastrozole, letrozole, vorazole or exemestane) or an inhibitor of 5$\alpha$-reductase such as finasteride;

(iii) an agent which inhibits cancer cell invasion (for example a metalloproteinase inhibitor like marimastat or an inhibitor of urokinase plasminogen activator receptor function);

(iv) an inhibitor of growth factor function, for example: a growth factor antibody (for example the anti-erbb2 antibody trastuzumab, or the anti-erbb1 antibody cetuximab [C225]), a farnesyl transferase inhibitor, a tyrosine kinase inhibitor or a serine/threonine kinase inhibitor, an inhibitor of the epidermal growth factor family (for example an EGFR family tyrosine kinase inhibitor such as N-(3-chloro-4-fluorophenyl)-7-methoxy-6-(3-morpholinopropoxy)quinazolin-4-amine (gefitinib, AZD1839), N-(3-ethynylphenyl)-6,7-bis(2-methoxyethoxy)quinazolin-4-amine (erlotinib, OSI-774) or 6-acrylamido-N-(3-chloro-4-fluorophenyl)-7-(3-morpholinopropoxy)quinazolin-4-amine (CI 1033)), an inhibitor of the platelet-derived growth factor family, or an inhibitor of the hepatocyte growth factor family;

(v) an antiangiogenic agent such as one which inhibits the effects of vascular endothelial growth factor (for example the anti-vascular endothelial cell growth factor antibody bevacizumab, a compound disclosed in WO 97/22596, WO 97/30035, WO 97/32856 or WO 98/13354), or a compound that works by another mechanism (for example linomide, an inhibitor of integrin $\alpha v\beta 3$ function or an angiostatin);

(vi) a vascular damaging agent such as combretastatin A4, or a compound disclosed in WO 99/02166, WO 00/40529, WO 00/41669, WO 01/92224, WO 02/04434 or WO 02/08213;

(vii) an agent used in antisense therapy, for example one directed to one of the targets listed above, such as ISIS 2503, an anti-ras antisense;

(viii) an agent used in a gene therapy approach, for example approaches to replace aberrant genes such as aberrant p53 or aberrant BRCA1 or BRCA2, GDEPT (gene-directed enzyme pro-drug therapy) approaches such as those using cytosine deaminase, thymidine kinase or a bacterial nitroreductase enzyme and approaches to increase patient tolerance to chemotherapy or radiotherapy such as multi-drug resistance gene therapy; or (ix) an agent used in an immunotherapeutic approach, for example ex-vivo and in-vivo approaches to increase the immunogenicity of patient tumour cells, such as transfection with cytokines such as interleukin 2, interleukin 4 or granulocyte-macrophage colony stimulating factor, approaches to decrease T-cell anergy, approaches using transfected immune cells such as cytokine-transfected dendritic cells, approaches using cytokine-transfected tumour cell lines and approaches using anti-idiotypic antibodies.

Some compounds of formula (I) may possess chiral centres. It is to be understood that the invention encompasses the use of all such optical isomers and diastereoisomers as well as compounds of formula (I) in any of these forms, and pharmaceutical compositions containing compounds of formula (I).

The invention further relates to all tautomeric forms of the compounds of formula (IA) and pharmaceutical compositions containing these.

It is also to be understood that certain compounds of the formula I can exist in solvated as well as unsolvated forms such as, for example, hydrated forms. It is to be understood that the invention encompasses all such solvated forms and pharmaceutical compositions containing these.

The compositions of the invention may be in a form suitable for oral use (for example as tablets, lozenges, hard or soft capsules, aqueous or oily suspensions, emulsions, dispersible powders or granules, syrups or elixirs), for topical use (for example as creams, ointments, gels, or aqueous or oily solutions or suspensions), for administration by inhalation (for example as a finely divided powder or a liquid aerosol), for administration by insufflation (for example as a finely divided powder) or for parenteral administration (for example as a sterile aqueous or oily solution for intravenous, subcutaneous, intermuscular or intramuscular dosing or as a suppository for rectal dosing).

The compositions of the invention may be obtained by conventional procedures using conventional pharmaceutical excipients, well known in the art. Thus, compositions intended for oral use may contain, for example, one or more colouring, sweetening, flavouring and/or preservative agents.

Suitable pharmaceutically acceptable excipients for a tablet formulation include, for example, inert diluents such as lactose, sodium carbonate, calcium phosphate or calcium carbonate, granulating and disintegrating agents such as corn starch or algenic acid; binding agents such as starch; lubricating agents such as magnesium stearate, stearic acid or talc; preservative agents such as ethyl or propyl p-hydroxybenzoate, and anti-oxidants, such as ascorbic acid.

Tablet formulations may be uncoated or coated either to modify their disintegration and the subsequent absorption of the active ingredient within the gastrointestinal tract, or to improve their stability and/or appearance, in either case, using conventional coating agents and procedures well known in the art.

Compositions for oral use may be in the form of hard gelatin capsules in which the active ingredient is mixed with an inert solid diluent, for example, calcium carbonate, calcium phosphate or kaolin, or as soft gelatin capsules in which the active ingredient is mixed with water or oil such as peanut oil, liquid paraffin, or olive oil.

Aqueous suspensions generally contain the active ingredient in finely powdered form together with one or more suspending agents, such as sodium carboxymethylcellulose, methylcellulose, hydroxypropylmethylcellulose, sodium alginate, polyvinyl-pyrrolidone, gum tragacanth and gum acacia; dispersing or wetting agents such as lecithin or condensation products of an alkylene oxide with fatty acids (for example polyoxyethylene stearate), or condensation products of ethylene oxide with long chain aliphatic alcohols, for example heptadecaethyleneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyethylene sorbitan monooleate. The aqueous suspensions may also contain one or more preservatives (such as ethyl or propyl p-hydroxybenzoate, anti-oxidants (such as ascorbic acid), colouring agents, flavouring agents, and/or sweetening agents (such as sucrose, saccharine or aspartame).

Oily suspensions may be formulated by suspending the active ingredient in a vegetable oil (such as arachis oil, olive oil, sesame oil or coconut oil) or in a mineral oil (such as liquid paraffin). The oily suspensions may also contain a thickening agent such as beeswax, hard paraffin or cetyl alcohol. Sweetening agents such as those set out above, and flavouring agents may be added to provide a palatable oral preparation. These compositions may be preserved by the addition of an anti-oxidant such as ascorbic acid.

Dispersible powders and granules suitable for preparation of an aqueous suspension by the addition of water generally contain the active ingredient together with a dispersing or wetting agent, suspending agent and one or more preservatives. Suitable dispersing or wetting agents and suspending agents are exemplified by those already mentioned above. Additional excipients such as sweetening, flavouring and colouring agents, may also be present.

The pharmaceutical compositions of the invention may also be in the form of oil-in-water emulsions. The oily phase may be a vegetable oil, such as olive oil or arachis oil, or a mineral oil, such as for example liquid paraffin or a mixture of any of these. Suitable emulsifying agents may be, for example, naturally-occurring gums such as gum acacia or gum tragacanth, naturally-occurring phosphatides such as soya bean, lecithin, and esters or partial esters derived from fatty acids and hexitol anhydrides (for example sorbitan monooleate) and condensation products of the said partial esters with ethylene oxide such as polyoxyethylene sorbitan monooleate. The emulsions may also contain sweetening, flavouring and preservative agents.

Syrups and elixirs may be formulated with sweetening agents such as glycerol, propylene glycol, sorbitol, aspartame or sucrose, and may also contain a demulcent, preservative, flavouring and/or colouring agent.

The pharmaceutical compositions may also be in the form of a sterile injectable aqueous or oily suspension, which may be formulated according to known procedures using one or more of the appropriate dispersing or wetting agents and suspending agents, which have been mentioned above. A sterile injectable preparation may also be a sterile injectable solution or suspension in a non-toxic parenterally-acceptable diluent or solvent, for example a solution in 1,3-butanediol.

Suppository formulations may be prepared by mixing the active ingredient with a suitable non-irritating excipient, which is solid at ordinary temperatures but liquid at the rectal temperature and will therefore melt in the rectum to release the drug. Suitable excipients include, for example, cocoa butter and polyethylene glycols.

Topical formulations, such as creams, ointments, gels and aqueous or oily solutions or suspensions, may generally be obtained by formulating an active ingredient with a conventional, topically acceptable, vehicle or diluent using conventional procedure well known in the art.

Compositions for administration by insufflation may be in the form of a finely divided powder containing particles of average diameter of, for example, 30$\mu$ or much less, the powder itself comprising either active ingredient alone or diluted with one or more physiologically acceptable carriers such as lactose. The powder for insufflation is then conveniently retained in a capsule containing, for example, 1 to 50mg of active ingredient for use with a turbo-inhaler device, such as is used for insufflation of the known agent sodium cromoglycate.

Compositions for administration by inhalation may be in the form of a conventional pressurized aerosol arranged to dispense the active ingredient either as an aerosol containing finely divided solid or liquid droplets. Conventional aerosol propellants such as volatile fluorinated hydrocarbons or hydrocarbons may be used and the aerosol device is conveniently arranged to dispense a metered quantity of active ingredient.

For further information on formulation the reader is referred to Chapter 25.2 in Volume 5 of Comprehensive Medicinal Chemistry (Corwin Hansch; Chairman of Editorial Board), Pergamon Press 1990.

The amount of active ingredient that is combined with one or more excipients to produce a single dosage form will necessarily vary depending upon the host treated and the particular route of administration. For example, a formulation

intended for oral administration to humans will generally contain, for example, from 0.5 mg to 2 g of active agent compounded with an appropriate and convenient amount of excipients, which may vary from about 5 to about 98 percent by weight of the total composition. Dosage unit forms will generally contain about 1 mg to about 500 mg of an active ingredient. For further information on Routes of Administration and Dosage Regimes the reader is referred to Chapter 25.3 in Volume 5 of Comprehensive Medicinal Chemistry (Corwin Hansch; Chairman of Editorial Board), Pergamon Press 1990.

The size of the dose for therapeutic or prophylactic purposes of a compound of the Formula (I) will naturally vary according to the nature and severity of the conditions, the age and sex of the animal or patient and the route of administration, according to well known principles of medicine.

In using a compound of the Formula (I) for therapeutic or prophylactic purposes it will generally be administered so that a daily dose in the range, for example, 0.5 mg to 75 mg per kg body weight is received, given if required in divided doses. In general lower doses will be administered when a parenteral route is employed. Thus, for example, for intravenous administration, a dose in the range, for example, 0.5 mg to 30 mg per kg body weight will generally be used. Similarly, for administration by inhalation, a dose in the range, for example, 0.5 mg to 25 mg per kg body weight will be used. Oral administration is however preferred.

The compounds of the present invention are useful in therapy, especially for the therapy of various pain conditions including, but not limited to: acute pain, chronic pain, neuropathic pain, back pain, cancer pain, and visceral pain. In a particular embodiment, the compounds are useful in therapy for neuropathic pain. In an even more particular embodiment, the compounds are useful in therapy for chronic neuropathic pain.

In another embodiment, the compounds of the present invention may be used to treat pain.

In another particular embodiment, the compounds of the present invention may be used to treat neuropathic pain.

Particularly, there is provided a pharmaceutical composition comprising a compound of Formula (I) or a pharmaceutically acceptable salt thereof, in association with a pharmaceutically acceptable carrier for therapy, more particularly for therapy of pain.

In use for therapy in a warm-blooded animal such as, for example, a human, the compound of the present invention may be administered in the form of a conventional pharmaceutical composition by any route including orally, intramuscularly, subcutaneously, topically, intranasally, intraperitoneally, intrathoracially, intravenously, epidurally, intrathecally, transdermally, intracerebroventricularly and by injection into the joints.

Within the scope of the invention is the use of any compound of Formula (I) as defined above for the manufacture of a medicament.

Also within the scope of the invention is the use of any compound of Formula (I) for the manufacture of a medicament for the therapy of pain.

Additionally provided is the use of any compound according to Formula (I) for the manufacture of a medicament for the therapy of various pain conditions including, but not limited to: acute pain, chronic pain, neuropathic pain, back pain, cancer pain, and visceral pain.

A further aspect of the invention is a method for therapy of a subject suffering from any of the conditions discussed above, whereby an effective amount of a compound according to the Formula (I) above, is administered to a patient in need of such therapy.

In a further aspect, the invention provides a method of treating inflammatory disease by administering a compound of Formula (I) as described above, or a pharmaceutical composition as described above.

Compounds of the invention having 2R stereochemistry (on the piperazine ring) are a particular aspect of this invention.

The invention will now be illustrated by the following non-limiting Examples in which, unless stated otherwise:

(i) temperatures are given in degrees Celsius (°C); operations were carried out at room or ambient temperature, that is, at a temperature in the range of 18-25 °C;

(ii) organic solutions were dried over anhydrous magnesium sulfate; evaporation of solvent was carried out using a rotary evaporator under reduced pressure (600-4000 Pascals; 4.5-30 mm Hg) with a bath temperature of up to 60 °C;

(iii) chromatography unless otherwise stated means flash chromatography on silica gel; thin layer chromatography (TLC) was carried out on silica gel plates (iv) in general, the course of reactions was followed by TLC and reaction times are given for illustration only;

(v) yields, when given, are for illustration only and are not necessarily those which can be obtained by diligent process development; preparations were repeated if more material was required;

(vi) when given, $^{1}H$ NMR data is quoted and is in the form of delta values for major diagnostic protons, given in parts per million (ppm) relative to tetramethylsilane (TMS) as an internal standard, determined at 400 MHz using perdeuterio DMSO ($CD_3SOCD_3$) as the solvent unless otherwise stated; coupling constants (J) are given in Hz;

(vii) chemical symbols have their usual meanings; SI units and symbols are used;

(viii) solvent ratios are given in percentage by volume;

(ix) mass spectra (MS) were run with an electron energy of 70 electron volts in the chemical ionisation (APCI) mode using a direct exposure probe; where indicated ionisation was effected by electrospray (ES); where values for m/z are given, generally only ions which indicate the parent mass are reported, and unless otherwise stated the mass ion quoted is the positive mass ion - $(M+H)^+$;

(x) LCMS characterisation was performed using a pair of Gilson 306 pumps with Gilson 233 XL sampler and Waters Micromass ZQ mass spectrometer. The LC comprised water symmetry 2x50 column C18 with 5 micron particle size. The eluents were: A, water with 0.1% formic acid and B, acetonitrile with 0.1% formic acid. The eluent gradient went from 95% A to 95% B in 5 minutes. Where indicated ionisation was effected by electrospray (ES); where values for m/z are given, generally only ions which indicate the parent mass are reported, and unless otherwise stated the mass ion quoted is the positive mass ion-$(M+H)^+$ and

(xi) the following abbreviations are used: DIPEA: diisopropylethylamine; DMSO: dimethyl sulfoxide; DMF: *N,N*-dimethylformamide; DCM: dichloromethane; EDCI:1-(3-dimethylaminopropyl)-3-ethylcarbodiimide hydrochloride; EtOAc: ethyl acetate; eq: equivalent; g: gram; $^1$H NMR: proton nuclear magnetic resonance; HATU: *O*-(7-azabenzotriazol-1yl)-*N,N,N',N'*-tetramethyluronium hexafluorophosphate; HOBt: 1-hydroxybenzotriazole; m: multiplet; M: molar; mL: milliliter; mg: milligram; MHz: megahertz; MeOH: methanol; RT: retention time (HPLC); s: singlet; SCX: Strong Cation Exchange; t: triplet; THF: tetrahydrofuran; TFA: trifluoroacetic acid; and TEA: triethylamine.

## Example 1

**4-[(2R)-4-tert-butylpiperazine-2-carbonyl]-N[3(trifluoromethyl)phenyl] piperazine-1-carboxamide**

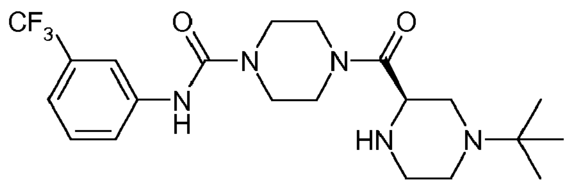

To a solution of tert-butyl (2R)-4-tert-butyl-2-(piperazine-1-carbonyl)piperazine-1-carboxylate (177 mg) in DCM (10 ml) was added, in one portion, 1-isocyanato-3-(trifluoromethyl)benzene (94 mg) and the resulting mixture was stirred for 1 hour. The reaction mixture was then purified by pouring onto a 20 g silica bond elut, eluting with a mixture of EtOAc/isohexane (50:50). The resulting product was dissolved in DCM (10 ml), TFA (2 ml) was added and stirred for 1 hour, then poured onto a 20 g SCX bond elut, eluting with MeOH then 10% 7M $NH_3$/MeOH in MeOH to give title compound (184 mg). $^1$H NMR (400 MHz, $CDCl_3$) $\delta$ ppm 1.1 (9H, s), 2.1-2.15 (2H, m), 2.9 (2H, m), 3.0-3.1 (2H, m), 3.4-3.7 (10H, m), 6.6 (1H, bs), 7.3 (1H, m), 7.35 (1H, m), 7.6 (1H, m), 7.6 (1H, s). LCMS *M*/z(+) 442 ($M+H^+$).

## Example 2

**4-[(2R)-4-tert-butylpiperazine-2-carbonyl]-N-(3-chlorophenyl)piperazine-1-carboxamide**

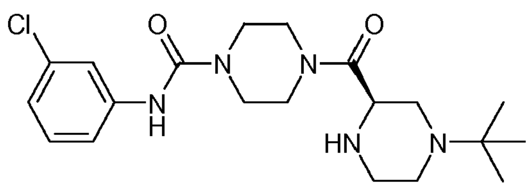

1-chloro-3-isocyanatobenzene (61 μL) was added in one portion to a solution of tert-butyl (2R)-4-tert-butyl-2-(piperazine-1-carbonyl)piperazine-1-carboxylate (177 mg) in DCM (5 ml). The resulting mixture was stirred at room temperature for 18 hours. The reaction mixture was poured onto a 50 g silica bond elut, eluting with EtOAc/isohexane (50:50). The resulting product was dissolved in DCM (10 ml), TFA (2 ml) was added, and the resulting mixture was stirred at room temperature for 2 hours. Poured onto a 20 g SCX bond elut, eluting with MeOH then 10% 7M $NH_3$/MeOH in MeOH to give title compound (159 mg). $^1$H NMR (400 MHz, $CDCl_3$) $\delta$ ppm 1.05 (9H, s), 2.0-2.15 (2H, m), 2.8-2.95 (2H, m), 3.0-3.15 (2H, m), 3.4-3.9 (9H, m), 6.5 (1H, bs), 7.0-7.05 (1H, m), 7.2 (2H, m), 7.5 (1H, m). LCMS *M*/z(+) 408

($M$+H$^+$).

## Example 3

**4-[(2R)-4-tert-butylpiperazine-2-carbonyl]-N-[3-(trifluoromethylsulfanyl)phenyl] piperazine-1-carboxamide**

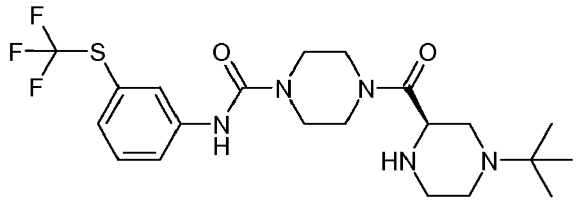

To a solution of 1-isocyanato-3-(trifluoromethylsulfanyl)benzene (111 mg) in THF (5 ml) was added tert-butyl (2R)-4-tert-butyl-2-(piperazine-1-carbonyl)piperazine-1-carboxylate (149 mg) and the resulting mixture was stirred at room temperature for 18 hours. The reaction mixture was evaporated to dryness and purified directly on 20 g Silicycle column bond elut, eluting with a gradient of MeOH /DCM (0-20%). The resulting product was dissolved in DCM (6 ml), TFA (2 ml) was added and the resulting mixture was stirred at room temperature until reaction had gone to completion. Poured directly onto a 20 g SCX bond elut, eluting with EtOAc then MeOH and then finally with 10% 7M $NH_3$/MeOH in MeOH to give title compound (161 mg). $^1$H NMR (400.13 MHz, DMSO-$d_6$) $\delta$ ppm 1.01 (9H, s), 1.94-2.04 (2H, m), 2.67-2.71 (1H, m), 2.80 (1H, d), 3.41 (1H, s), 3.46 (1H, s), 3.50 (1H, s), 3.55 (4H, d), 3.69 (2H, d), 7.27 (1H, d), 7.41 (1H, t), 7.68-7.71 (1H, m), 7.93 (1H, s), 8.85 (1H, s). LCMS $M$/z(+) 474 ($M$+H$^+$).

## Example 4

**N-(4-bromophenyl)-4-[(2R)-4-tert-butylpiperazine-2-carbonyl]piperazine-1-carboxamide**

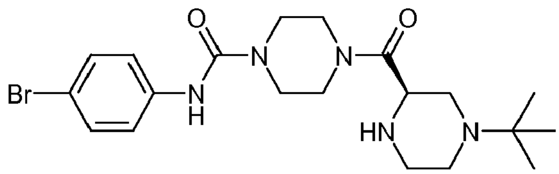

To a solution of 1-bromo-4-isocyanatobenzene (153 mg) in THF (5 ml) was added tert-butyl (2R)-4-tert-butyl-2-(piperazine-1-carbonyl)piperazine-1-carboxylate (227 mg) and the resulting mixture was stirred at room temperature for 18 hours. The reaction mixture was evaporated to dryness and purified directly on 20 g Silicycle column eluting with a gradient of MeOH/DCM (0-20%). The resulting product was dissolved in DCM (6ml), TFA (2 ml) was added and the resulting mixture was stirred at room temperature until reaction had gone to completion. Poured directly onto a 20 g SCX bond elut, eluting with EtOAc then MeOH and then finally with 10% 7M $NH_3$/MeOH in MeOH to give title compound (68 mg). $^1$H NMR (400.13 MHz, DMSO-$d_6$) $\delta$ ppm 1.00-1.02 (9H, m), 2.83 (1H, d), 2.99 (1H, d), 3.38 (1H, s), 3.44-3.48 (2H, m), 3.49-3.52 (2H, m), 3.56-3.60 (3H, m), 3.75 (1H, d), 5.75 (3H, s), 7.41 (1H, q), 7.42-7.45 (2H, m), 7.45-7.47 (1H, m), 8.70 (1H, s). LCMS $M$/z(+) 454 ($M$+H$^+$).

## Example 5

**4-[(2R)-4-tert-butylpiperazine-2-carbonyl]-N-[4-fluoro-3(trifluoromethyl) phenyl] piperazine-1-carboxamide**

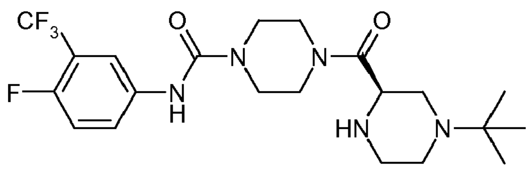

To a solution of 1-fluoro-4-isocyanato-2-(trifluoromethyl)benzene (99 mg) in THF (5 ml) was added tert-butyl (2R)-4-tert-butyl-2-(piperazine-1-carbonyl)piperazine-1-carboxylate (142 mg) and the resulting mixture was stirred at room temperature for 18 hours. The reaction mixture was evaporated to dryness and purified directly on 20 g Silicycle column eluting with a gradient of MeOH/DCM (0-20%). The resulting product was dissolved in DCM (6 ml), TFA (2 ml) was added and the reaction mixture was stirred at room temperature until reaction had gone to completion. Poured directly onto a 20 g SCX bond elut, eluting with EtOAc then MeOH and then finally with 10% 7M $NH_3$/MeOH in MeOH to give title compound (22 mg). $^1H$ NMR (400.13 MHz, DMSO-$d_6$) δ ppm 1.01 (9H, s), 1.98 (1H, d), 2.02 (1H, s), 2.67 (1H, d), 2.96 (1H, d), 3.40 (1H, s), 3.46 (2H, s), 3.54 (4H, s), 3.69 (2H, d), 5.75 (2H, s), 7.41 (1H, d), 7.77-7.80 (1H, m), 7.92-7.94 (1H, m), 8.91 (1H, s). LCMS *M*/z(+) 460 (*M*+$H^+$).

## Example 6

**4-[(2R)-4-tert-butylpiperazine-2-carbonyl]-N-[4-methyl-3-(trifluoromethyl) phenyl]piperazine-1-carboxamide**

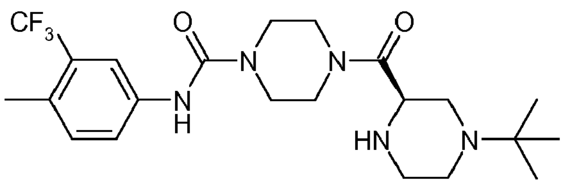

To a solution of tert-butyl (2R)-4-tert-butyl-2-(piperazine-1-carbonyl)piperazine-1-carboxylate (227 mg) in THF (5 ml) was added 4-isocyanato-1-methyl-2-(trifluoromethyl)benzene (120 μl) and the resulting mixture was stirred at room temperature. The reaction mixture was evaporated and purified by silica chromatography (40 g Redisep) eluting with a gradient of EtOAc/isohexane (50-100%). The resulting product was dissolved in DCM (6 ml), TFA (2 ml) was added and the resulting mixture was stirred at room temperature for 1 hour. The reaction mixture was poured directly onto a 20 g SCX bond elut, and eluting with EtOAc then MeOH followed by 10% 7M $NH_3$/MeOH in MeOH to give the crude title compound which was further purified by silica chromatography eluting with a gradient of MeOH/DCM (0-70%) to give title compound (170 mg). $^1H$ NMR (400.13 MHz, DMSO-$d_6$) δ ppm 1.00 (9H, s), 1.97-2.03 (2H, m), 2.36 (3H, d), 2.63-2.70 (1H, m), 2.80 (1H, d), 3.41-3.45 (2H, m), 3.51 (1H, s), 3.53-3.60 (4H, m), 3.69 (1H, d), 7.30 (1H, d), 7.63-7.65 (1H, m), 7.86 (1H, d), 8.83 (1H, s). LCMS *M*/z(+) 456 (*M*+$H^+$).

## Example 7

**4-[(2R)-4-tert-butylpiperazine-2-carbonyl]-N-(3-chloro-4-fluorophenyl) piperazine-1-carboxamide**

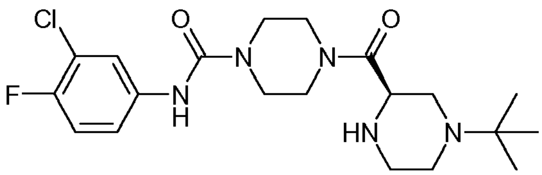

To a solution of tert-butyl (2R)-4-tert-butyl-2-(piperazine-1-carbonyl)piperazine-1-carboxylate (50 mg) in DCM (5 ml) was added a solution of the 2-chloro-1-fluoro-4-isocyanatobenzene (25 mg). The mixture was stirred for 2 hours then concentrated under reduced pressure. The residue was dissolved in DCM (10 mL), TFA (5 mL) was added and the resulting mixture was stirred at room temperature for 2 hours. The reaction mixture was concentrated at reduced

pressure. Loaded onto an SCX-2 column, eluted with MeOH, then with 10% 7M $NH_3$/MeOH in MeOH, which was repurified by silica column chromatography (12 g column) eluting with a gradient of MeOH in DCM (0-25%) to give title compound (58 mg). $^1$H NMR (400.132 MHz, $CDCl_3$) δ ppm 1.07 (9H, s), 2.02-2.17 (2H, m), 2.83-2.95 (2H, m), 3.02 (1H, d), 3.06-3.13 (1H, m), 3.36-3.89 (10H, m), 6.36 (1H, s), 7.05 (1H, t), 7.18 (1H, ddd), 7.51 (1H, dd). LCMS *M*/z(+) 423.9 & 425.9 (*M*+$H^+$ Cl pattern).

**Example 8**

**4-[(2R)-4-tert-butylpiperazine-2-carbonyl]-N-[4-cyano-3-(trifluoromethyl) phenyl]piperazine-1-carboxamide**

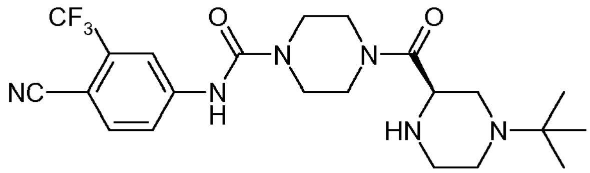

To a solution of tert-butyl (2R)-4-tert-butyl-2-(piperazine-1-carbonyl)piperazine-1-carboxylate (231 mg) in DCM (5 ml) was added TEA (272 μL) followed by phenyl N-[4-cyano-3-(trifluoromethyl)phenyl]carbamate (199 mg). The resulting mixture was heated to 60 °C for 1 hour. Cooled to room temperature, washed with water, then brine, dried ($MgSO_4$) and concentrated under reduced pressure. The resulting residue was purified on a silica bond elut, eluting with EtOAc/isohexane (50:50) followed by a gradient of MeOH in EtOAc (0-20%). The resulting product was dissolved in DCM (10ml), TFA (2ml) was added and the resulting mixture was stirred at room temperature for 1.5 hours. Poured onto SCX bond elut, eluting with MeOH then 10% 7M $NH_3$/MeOH in MeOH to give title compound (210 mg). $^1$H NMR (400 MHz, $CDCl_3$) δ ppm 1.1 (9H, s), 1.8 (1H, bs), 2.10-2.25 (2H, m), 2.85-3.35 (4H, m), 3.4-3.9 (9H, m), 7.35 (1H, bs), 7.7 (1H, d), 7.8 (1H, dd), 7.9 (1H, d). LCMS *M*/z(+) 467 (M+$H^+$).

**Example 9**

**4-[(2R)-4-tert-butylpiperazine-2-carbonyl]-N-(4-chloro-3-fluorophenyl) piperazine-1-carboxamide**

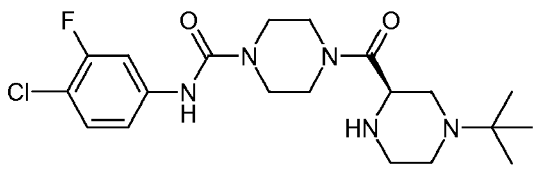

To a solution of tert-butyl (2R)-4-tert-butyl-2-(piperazine-1-carbonyl)piperazine-1-carboxylate (354 mg) in DCM (15 ml) was added TEA (0.42 ml) followed by phenyl N-(4-chloro-3-fluorophenyl)carbamate (265 mg). The resulting mixture was heated to 60 °C for 1 hour. Cooled to room temperature, concentrated under reduced pressure and the residue was purified on a silica bond elut, eluting with a gradient of EtOAc/isohexane (50-100%). The resulting product was dissolved in DCM (10 ml), TFA (2 ml) was added and the resulting mixture was stirred at room temperature for 2 hours and then poured onto a SCX bond elut, eluting with MeOH then 10% 7M $NH_3$/MeOH in MeOH to give title compound (286 mg). $^1$H NMR (400MHz, $CDCl_3$) δ ppm 1.1 (9H, s), 2.05-2.15 (2H, m), 2.8-3.15 (4H, m), 3.4-3.8 (8H, m), 6.55 (1H, bs), 7.0 (1H, m), 7.2 (1H, m), 7.4 (1H, m). LCMS *M*/z(+) 426 (*M*+$H^+$).

**Example 10**

**4-[(2R)-4-tert-butylpiperazine-2-carbonyl]-N-[3-(1,1,2,2-tetrafluoroethoxy) phenyl]piperazine-1-carboxamide**

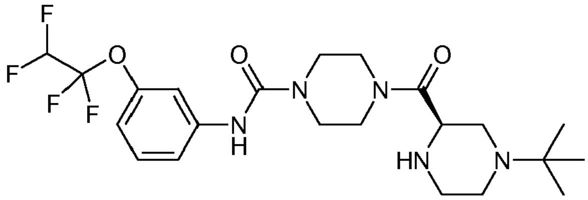

To a solution of tert-butyl (2R)-4-tert-butyl-2-(piperazine-1-carbonyl)piperazine-1-carboxylate (354 mg) in DCM (15 ml) was added TEA (0.42 ml) followed by phenyl N-[3-(1,1,2,2-tetrafluoroethoxy)phenyl]carbamate (329 mg). The resulting mixture was heated to 60 °C for 1 hour, cooled to room temperature and concentrated under reduced pressure. The residue was purified on a silica bond elut, eluting with a gradient of EtOAc/isohexane (50-100%). The resulting product was dissolved in DCM (10 ml), TFA (2 ml) was added and the resulting mixture was stirred at room temperature for 2 hours. The reaction mixture was poured onto a SCX bond elut, eluting with MeOH then 10% 7M $NH_3$/MeOH in MeOH to give title compound (448 mg). $^1H$ NMR (400MHz, $CDCl_3$) $\delta$ ppm 1.0 (9H, s), 2.0-2.15 (2H, m), 2.8-2.9 (2H, m), 3.0 (1H, m), 3.1 (1H, m), 3.4-3.85 (9H, m), 5.7 and 5.85 and 6.0 (1H, m), 6.45 (1H, bs), 6.9 (1H, m), 7.2-7.25 (2H, m), 7.3 (1H, s). LCMS *M*/z(+) 490 (*M*+$H^+$).

## Example 11

**4-[(2R)-4-tert-butylpiperazine-2-carbonyl]-N-(4-chloro-3-methylphenyl) piperazine-1-carboxamide**

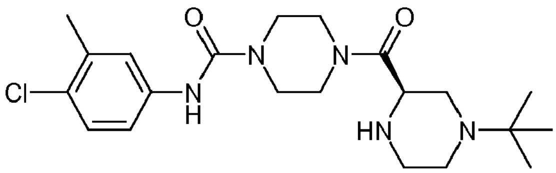

To a solution of tert-butyl (2R)-4-tert-butyl-2-(piperazine-1-carbonyl)piperazine-1-carboxylate (177 mg) in DCM (5 ml) was added TEA (210 $\mu$L) followed by phenyl N-(4-chloro-3-methylphenyl)carbamate (131 mg). The resulting mixture was heated to 60 °C for 1 hour, cooled to room temperature, washed with water, then brine, dried ($MgSO_4$) and concentrated *in vacuo.* The residue was purified on a silica bond elut, eluting with a gradient of EtOAc/isohexane (20-100%). The resulting product was dissolved in DCM (10 ml), TFA (2 ml) was added, and the resulting mixture was stirred at room temperature for 18 hours. The reaction mixture was poured onto SCX bond elut, eluting with MeOH then 10% 7M $NH_3$/MeOH in MeOH to give title compound (132 mg). $^1H$ NMR (400MHz, $CDCl_3$) $\delta$ ppm 1.0 (9H, s), 1.95-2.05 (2H, m), 2.25 (3H, s), 2.75-3.15 (4H, m), 3.3-3.8 (9H, m), 6.3 (1H, bs), 7.0-7.1 (1H, m), 7.15-7.25 (2H, m). LCMS *M*/z(+) 422 (*M*+$H^+$).

## Example 12

**4-[(2R)-4-tert-butylpiperazine-2-carbonyl]-N-(3-phenylmethoxyphenyl) piperazine-1-carboxamide**

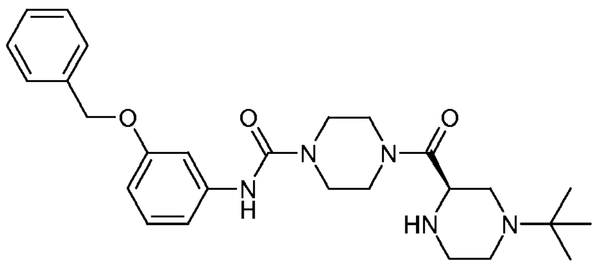

To a solution of tert-butyl (2R)-4-tert-butyl-2-(piperazine-1-carbonyl)piperazine-1-carboxylate (354 mg) in DCM

(15 ml) was added TEA (0.42 ml) followed by phenyl N-(3-phenylmethoxyphenyl)carbamate (159.5 mg). The resulting mixture was heated to 60 °C for 1 hour, cooled to room temperature and purified on a silica bond elut, eluting with a gradient of EtOAc/isohexane (50-100%). The resulting product was dissolved in DCM (10 ml), TFA (2 ml) was added, and the resulting mixture was stirred at room temperature for 18 hours. The reaction mixture was poured onto a SCX bond elut, eluting with MeOH then 10% 7M $NH_3$/MeOH in MeOH to give title compound (40 mg). $^1$H NMR (400MHz, $CDCl_3$) δ ppm 1.0 (9H, s), 2.0-2.1 (2H, m), 2.8 (2H, m), 2.9-3.0 (2H, m), 3.3-3.8 (10H, m), 4.9 (2H, s), 6.35 (1H, bs), 6.6 (1H, d), 6.75 (1H, d), 7.0-7.10 (2H, m), 7.15-7.3 (5H, m). LCMS *M*/z(+) 480 (*M*+H$^+$).

## Example 13

**4-[(2R)-4-tert-butylpiperazine-2-carbonyl]-N-[3-chloro-4-(trifluoromethoxy) phenyl]piperazine-1-carboxamide**

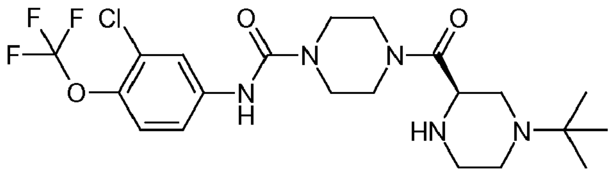

To a solution of tert-butyl (2R)-4-tert-butyl-2-(piperazine-1-carbonyl)piperazine-1-carboxylate (1.775 g) in DCM (25 ml) was added TEA (2.095 ml) followed by phenyl N-[3-chloro-4-(trifluoromethoxy)phenyl]carbamate (780 mg). The resulting mixture was heated to 60 °C for 1 hour. Cooled to room temperature, washed with water, then brine, dried ($MgSO_4$) and concentrated under reduced pressure. The residue was purified on a silica bond elut, eluting with EtOAc/isohexane (50:50) followed by a gradient of MeOH in EtOAc (0-20%). The resulting product was dissolved in DCM (10 ml), TFA (2 ml) was added and the resulting mixture was stirred at room temperature for 18 hours. The reaction mixture was poured onto SCX bond elut, eluting with MeOH then 10% 7M $NH_3$/MeOH in MeOH to give title compound (1.3 g). $^1$H NMR (400MHz, $CDCl_3$) δ ppm 1.05 (9H, s), 2.10-2.30 (2H, m) 2.85-3.20 (4H, m) 3.40-3.95 (9H, m) 6.95 (1H, s) 7.20 (1H, d) 7.30 (1H, d) 7.65 (1H, d). LCMS *M*/z(+) 492 (*M*+H$^+$).

## Example 14

**N-(5-bromo-4-methyl-1,3-thiazol-2-yl)-4-[(2R)-4-tert-butylpiperazine-2-carbonyl]piperazine-1-carboxamide.**

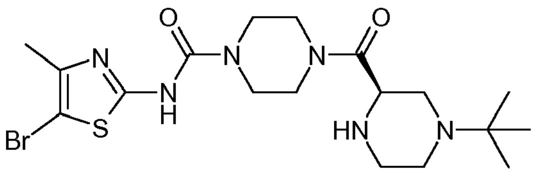

To a solution of tert-butyl (2R)-4-tert-butyl-2-(piperazine-1-carbonyl)piperazine-1-carboxylate (177 mg) in DCM (5 ml) was added TEA (91 μl) followed by phenyl N-(5-bromo-4-methyl-1,3-thiazol-2-yl)carbamate (64 mg) and the resulting mixture was stirred and heated to 60 °C for 2 hours. Cooled to room temperature and purified on a 20 g silica bond elut, eluting with ethyl hexane/isohexane (50:50). The resulting product was dissolved in DCM (5 ml), treated with TFA (1 ml) and the resulting mixture was stirred for 18 hours. The reaction mixture was poured onto an SCX bond elut, eluted with MeOH and then 10% 7M $NH_3$/MeOH in MeOH to give title compound (151 mg). $^1$H NMR (400MHz, $CDCl_3$) δ ppm 1.0 (9H, m), 2.0-2.1 (2H, m), 2.15 (3H, s), 2.8-2.85 (2H, m), 2.9-2.05 (2H, m), 3.3-3.8 (9H, m). LCMS *M*/z(+) 475(*M*+H$^+$).

## Example 15

**4-[(2R)-4-tert-butylpiperazine-2-carbonyl]-N-(5-chloro-4-ethyl-1,3-thiazol-2-yl)piperazine-1-carboxamide**

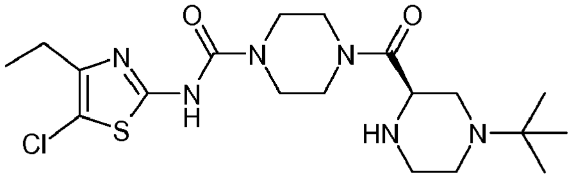

N-(5-chloro-4-ethyl-1,3-thiazol-2-yl)piperazine-1-carboxamide (398 mg) was added to a stirred solution of (2R)-4-tert-butyl-1-[(2-methylpropan-2-yl)oxycarbonyl]piperazine-2-carboxylic acid (416 mg) in THF (10 ml). HOBT (222 mg), EDCI (277 mg) and DIPEA (253 μl) were then added and the resulting mixture was stirred at room temperature for 18 hours and concentrated under reduced pressure. The residue was dissolved in DCM, washed with 2M NaOH, and brine then dried ($MgSO_4$) and concentrated *in-vacuo.* The residue was purified by silica chromatography eluting with EtOAc/iso-Hexane (50:50) and the resulting product was dissolved in DCM (10 ml), TFA (2 ml) was added and the resulting mixture was stirred for 2 hours. The reaction mixture was poured onto an SCX bond elut and eluted with MeOH and then 10% 7M $NH_3$/MeOH in MeOH to give title compound (280 mg). $^1H$ NMR (400MHz, $CDCl_3$) δ ppm 1.05 (6H, dd), 1.15-1.25 (3H, m), 2.1-2.3 (2H, m), 2.55-2.65 (2H, m), 2.7-2.8 (2H, m), 2.85-3.0 (2H, m), 3.05-3.15 (1H, m), 3.5-3.8 (9H, m), 3.85-3.90 (1H, m). LCMS *M*/z(+) 443 (*M*+$H^+$).

**Example 16**

**4-[(2R)-4-tert-butylpiperazine-2-carbonyl]-N-(5-chloro-4-methyl-1,3-thiazol-2-yl)piperazine-1-carboxamide**

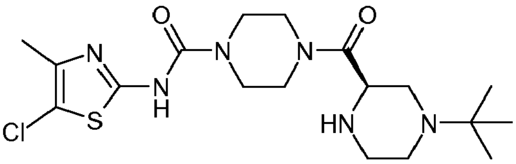

To a solution of tert-butyl (2R)-4-tert-butyl-2-(piperazine-1-carbonyl)piperazine-1-carboxylate (330 mg) in THF (10 ml) was added TEA (195 μl) followed by phenyl N-(5-chloro-4-methyl-1,3-thiazol-2-yl)carbamate (330 mg) and the resulting mixture was heated in a Microwave oven (Biotage *initiator)* at 60 °C for 1.5 hours. The mixture was concentrated and the resulting product was dissolved in DCM (20 ml), to which TFA (10 ml) was added and the resulting mixture was stirred at room temperature for 1.5 hours. The reaction mixture was poured onto a SCX bond elut and eluting with MeOH followed by 10% 7M $NH_3$/MeOH in MeOH, followed by a reverse phase chromatography to give title compound (235 mg). $^1H$ NMR (400MHz, $CDCl_3$) δ ppm 1.07 (9H, s), 2.01-2.18 (2H, m), 2.22 (3H, s), 2.83-3.13 (4H, m), 3.35-3.89 (10H, m). LCMS *M*/z(+) 429 (*M*+$H^+$).

**Example 17**

**4-[(2R)-4-tert-butylpiperazine-2-carbonyl]-N-[5-chloro-4-(trifluoromethyl)-1,3-thiazol-2-yl]piperazine-1-carboxamide**

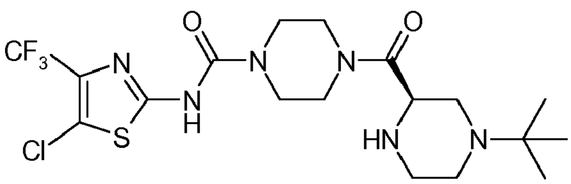

To a solution of tert-butyl (2R)-4-tert-butyl-2-(piperazine-1-carbonyl)piperazine-1-carboxylate (340 mg) in THF (10 ml) was added TEA (201 μl) followed by phenyl N-[5-chloro-4-(trifluoromethyl)-1,3-thiazol-2-yl]carbamate (310 mg) and the resulting mixture was heated in a microwave oven (Biotage *initiator)* at 60 °C for 1.5 hours. The reaction mixture was cooled and concentrated. The residue was dissolved in DCM (10 ml), TFA (5ml) was added and the resulting mixture

was stirred at room temperature for 1 hour. The reaction mixture was concentrated, dissolved in MeOH and poured onto an SCX column. Elution with MeOH followed by 10% 7M $NH_3$/MeOH in MeOH gave crude product which was further purified using silica chromatography eluting with a gradient of MeOH/DCM (0-25%) to afford title compound (415 mg). $^1$H NMR (400MHz, $CDCl_3$) δ ppm 1.06 (9H, s), 2.02-2.15 (2H, m), 2.82-2.94 (2H, m), 3.00 (1H, d), 3.06-3.13 (1H, m), 3.37-3.95 (10H, m). LCMS *M*/z(+) 483 (*M*+H$^+$).

**INTERMEDIATES**

**1: Preparation of Phenyl carbamates** **Phenyl N-(4-chloro-3-methylphenyl)carbamate**

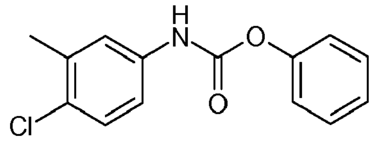

To a solution of 4-chloro-3-methylaniline (2 g) in DCM (30 ml), cooled to 0 °C, was added pyridine (2.285 ml) followed by phenyl chloroformate (1.85 ml) and the resulting mixture was stirred at room temperature for 18 hours. 1M HCl (20 ml) was added and stirred for 10 minutes. The organic layer was washed with water and then brine. The organic extracts were dried ($MgSO_4$), and evaporated to give a gum. The solid was slurried in hexane and filtered to give the titled compound as a pale brown solid. $^1$H NMR (400 MHz, $CDCl_3$) δ ppm 2.35 (3H, s), 7.15-7.40 (8H, m). LCMS *M*/z(+) 260 (*M*-H$^+$). In a similar manner, but using the appropriate aniline the following carbamates were prepared:

**Phenyl N-[4-cyano-3-(trifluoromethyl)phenyl]carbamate**

$^1$H NMR (300 MHz) δ ppm 7.2 (2H, m), 7.3 (1H, m), 7.4 (2H, m), 7.75 (2H, m), 7.95 (1H, d). LCMS *M*/z(+) 305 (M-H$^+$).

**Phenyl N-(4-chloro-3-fluorophenyl)carbamate**

$^1$H NMR (400 MHz, DMSO) δ ppm 6.85-7.4 (9H, m). LCMS *M*/z(+) 266 (*M*+H$^+$).

**Phenyl N-[3-(1,1,2,2-tetrafluoroethoxy)phenyl]carbamate**

$^1$H NMR (400.132 MHz, $CDCl_3$) δ ppm 5.75 - 6.04 (1H, m), 6.95 - 7.04 (2H, m), 7.16 - 7.51 (8H, m). LCMS *M*/z(+) 328 (*M*-H$^+$).

**Phenyl N-(3-phenylmethoxyphenyl)carbamate**

$^1$H NMR (400MHz) δ ppm 5.05 (2H, s), 6.7 (1H, m), 6.9 (2H, m), 7.15-7.40 (12H, m). LCMS *M*/z(+) 320 (*M*+H$^+$).

**Phenyl N-[3-chloro-4-(trifluoromethoxy)phenyl]carbamate**

$^1$H NMR (400.132 MHz, $CDCl_3$) δ ppm 7.00 (1H, s), 7.15 - 7.20 (2H, m), 7.23 - 7.43 (5H, m), 7.68 (1H, d). LCMS *M*/z(+) 330 (M-H$^+$).

**Phenyl N-(5-bromo-4-methyl-1,3-thiazol-2-yl)carbamate**

$^1$H NMR (400.132 MHz, DMSO) δ ppm 2.2 (3H, s) 7.2-7.3 (3H, m) 7.4 (2H, m) 12.3 (1H, bs). LCMS *M*/z(+) 314 (M+H$^+$).

**Phenyl N-[5-chloro-4-(trifluoromethyl)-1,3-thiazol-2-yl]carbamate**

$^1$H NMR (400.132 MHz, $CDCl_3$) δ 7.2 (2H, m), 7.25-7.35 (1H, m), 7.4 (2H, m). LCMS *M*/z(+) 322 (*M*+H$^+$).

**Phenyl N-(5-chloro-4-methyl-1,3-thiazol-2-yl)carbamate**

LCMS *M*/z(+) 269 (*M*+H+).

**2: Preparation of tert-butyl (2R)-4-tert-butyl-2-(piperazine-1-carbonyl) piperazine-1-carboxylate (Method A)**

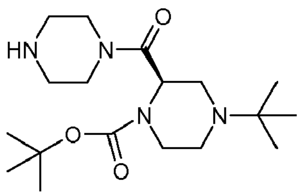

**Step1: preparation of 1-acetyl-4-benzylpiperazine**

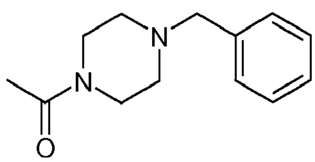

A solution of 1-benzylpiperazine (1 g) and triethylamine (1.19 mL) in THF (20 mL) was stirred at room temperature under argon. Acetyl chloride (0.424 mL) was added and the reaction mixture was stirred for 10 minutes. The reaction was filtered and the white solid washed with ether. The filtrate was concentrated at reduced pressure to give the title compound (1.2 g). LCMS *M*/z(+) 219.07 (*M*+H+).

**Step 2: preparation of 1-Benzyl-4-*tert*-butylpiperazine**

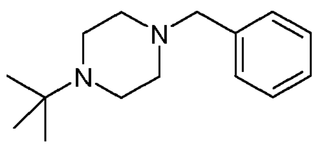

A solution of 1-acetyl-4-benzylpiperazine (1.2 g) in THF was stirred at -10 °C under argon. A 1M solution of titanium (V) chloride (1.2 mL) was then added and the mixture was stirred for 30 minutes. A 3M solution of methylmagnesium bromide in ether (11.3 mL) was then added dropwise and the black reaction mixture was warmed to ambient temperature and stirred overnight. The reaction mixture was quenched with 30% aqueous sodium hydroxide solution and then partitioned between water and dichloromethane. The layers were separated and the organic layer washed with brine, dried ($Na_2SO_4$), filtered and concentrated. The residue was purified on the Isco™ Companion (40g column: 0-10% MeOH in DCM) to give the title compound (770 mg). LCMS *M*/z(+) 233.09 (*M*+H+).

**Step 3: Preparation of *tert*-Butyl 4-*tert*-butylpiperazine-1-carboxylate**

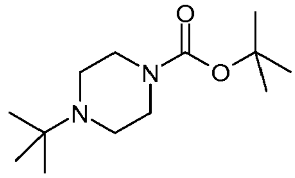

A mixture of 1-benzyl-4-*tert*-butylpiperazine (740 mg), di-tert-butyl dicarbonate (1.48 g) and 10% palladium on carbon (200 mg) in ethanol (10 mL) was evacuated and purged with hydrogen three times and then left under an atmosphere of hydrogen overnight at room temperature. The mixture was filtered through a short pad of celite and concentrated at reduced pressure. The residue was purified on the Isco™ Companion (40g column: 0-10% MeOH in DCM) to give the title compound (667mg). LCMS *M*/z(+) 243.09 ($M$+$H^+$).

**Step 4: Preparation of 1-(*tert*-Butoxycarbonyl)-4-*tert*-butylpiperazine-2-carboxylic acid**

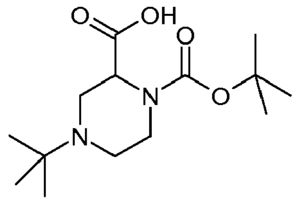

A solution of *tert*-butyl 4-*tert*-butylpiperazine-1-carboxylate (500 mg) and *N,N,N'N'*-tetramethylethylenediamine (0.467 mL) in ether (4 mL) was stirred at -78 °C under argon. A 1.4 M solution of sec-butyl lithium in cyclohexane (2.2 mL) was added dropwise and the mixture was stirred at -78 °C for 3.5 hours. Carbon dioxide was then bubbled through the reaction mixture via an argon purged syringe for 15 minutes at -78 °C and then whilst warming to 0 °C. The reaction was quenched by the addition of water and then diluted with dichloromethane, dried ($Na_2SO_4$), filtered and concentrated at reduced pressure. The residue was purified on the Isco™ Companion (40g column: 0-20% MeOH in DCM) to give the title compound (370 mg).
LCMS *M*/z(+) 286.99 ($M$+$H^+$).

**Step 5: Preparation of tert-butyl-2-(4-benzylpiperazine-1-carbonyl)-4-tert-butylpiperazine-1-carboxylate**

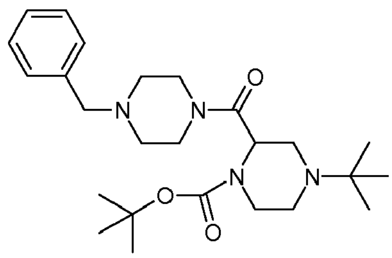

To a solution of 1-(*tert*-Butoxycarbonyl)-4-*tert*-butylpiperazine-2-carboxylic acid (4.6 g), 1-benzylpiperazine (3.5 ml) and DIPEA (5.6 ml) in DMF (15 ml) at room temperature was added HATU (6.1 g). After 2 hours water was added and the mixture was stirred for 2 hours. The mixture was then partitioned between ethyl acetate and water and the organic layer washed with brine (x2), dried ($Na_2SO_4$) and concentrated at reduced pressure. The residue was purified via silica column chromatography (120 g column) on the Isco companion eluting with a gradient methanol in ethyl acetate (0 to 5%) to give title compound (4.5 g). LCMS *M*/z(+) 445 ($M$+$H^+$).
The (R) and (S) isomers are readily separable - see Step 4, Method B and debenzylated to give the title compound (Step 5, Method B).

**3: Preparation of tert-butyl (2R)-4-tert-butyl-2-(piperazine-1-carbonyl) piperazine-1-carboxylate (Method B)**

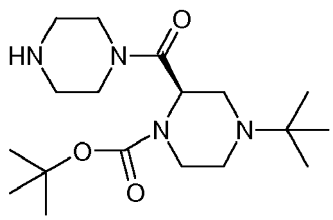

**Step 1: Preparation of *tert*-butyl 4-(2-cyanopropan-2-yl)piperazine-1-carboxylate**

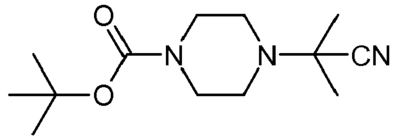

Potassium cyanide (25.0 g, 1.0 eq) was added in one go to a solution of *N*-Boc-piperazine (71.5 g, 1.0 eq) and *para*-toluenesulphonic acid monohydrate (24.7 g, 1.0 eq) in water (715 ml) at 25 °C. Acetone (282 ml, 10 eq) was then added and the reaction mixture was stirred at room temperature for 18 hours. The reaction mixture was partitioned between saturated aqueous sodium hydrogen carbonate solution (1000 ml) and ethylacetate (700 ml). The aqueous layer was separated and extracted with ethylacetate (2 x 700 ml), the organics were combined, dried ($MgSO_4$), filtered and the solvent removed *in vacuo.* The residue was purified by silica chromatography eluting with a gradient of methanol in DCM (0 to 10%) to afford the title compound (52 g) as a white solid.
$^1H$ NMR (400 MHz, $CDCl_3$) δ ppm 1.5 (9H, s), 1.55 (6H, s), 2.65 (4H, m), 3.5 (4H, m).

**Step 2: Preparation of *tert*-Butyl 4-*tert*-butylpiperazine-1-carboxylate**

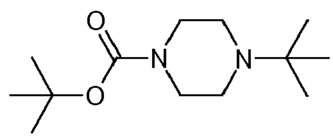

Methylmagnesiumbromide (3M in diethylether) (317 ml, 3.0 eq) was added dropwise over 30 minutes to a solution of *tert*-butyl 4-(2-cyanopropan-2-yl)piperazine-1-carboxylate (80 g, 1.0 eq) in THF (800 ml) at 15 °C, exotherm seen 15 °C - 28 °C. The reaction mixture was left stirred at room temperature for 18 hours. The reaction was cooled to -10 °C and water (800 ml) was added dropwise over 60 minutes (very exothermic -10 °C - 25 °C). Dichloromethane (1600 ml) was added and the organic layer was separated and the aqueous layer extracted with dichloromethane (1600 ml). The organics were combined and washed with 50% brine / water (1600 ml), dried ($MgSO_4$), filtered and the solvent removed *in vacuo.* The residue was purified by silica chromatography eluting with ethyl acetate to afford the title compound (48 g) as yellow solid.
$^1H$ NMR (400.13 MHz, DMSO-$d_6$) δ ppm 1.00 (9H, s), 1.4 (9H, s), 2.4 (4H, m), 3.3 (4H, m).

**Step 3: Preparation of (2R)-4-tert-butyl-1-[(2-methylpropan-2-yl)oxycarbonyl] piperazine-2-carboxylic acid**

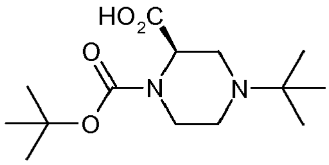

sec-Butyllithium (1.4 M in cyclohexane) (38.3 ml, 1.3 eq) was added dropwise over 20 minutes to a stirred solution of *tert*-butyl 4-*tert*-butylpiperazine-1-carboxylate (10 g, 1.0 eq) and (-)-sparteine (12.3 ml, 1.3 eq) in diethylether (300 ml) at -78 °C (acetone / solid carbon dioxide bath) under an atmosphere of nitrogen, keeping the internal temperature below -70 °C, the reaction was left stirring for 4 hours at -78 °C before carbon dioxide gas was bubbled through the reaction (exothermic -78 °C - -66 °C) for 1 hour (using dry ice). The reaction was allowed to warm to room temperature and stirred for 18 hours. Water (10ml) was then added dropwise to quench the reaction (no exotherm seen). Dichloromethane (200 ml) was added to dilute followed by sodium sulphate. The reaction was stirred for 10 minutes then filtered washing through with dichloromethane (200 ml) (slow filtration). The solvent was then removed *in vacuo* to give the crude carboxylic acid (24.5 g), which used in the next step without further purification. (Chiral analysis of crude material showed compound to have ~65%ee).

**Step 4: preparation of tert-butyl (2R)-2-(4-benzylpiperazine-1-carbonyl)-4-tert-butylpiperazine-1-carboxylate**

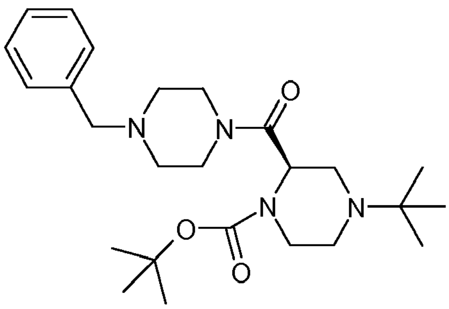

Diisopropylethylamine (14.7 ml, 2.0 eq) was added dropwise to a solution of crude (step 3) (2R)-4-tert-butyl-1-[(2-methylpropan-2-yl)oxycarbonyl]piperazine-2-carboxylic acid (37.1 g), N-benzylpiperazine (9 ml, 1.25 eq) and HATU (15.7 g, 1.0 eq) in DMF (59 ml) at room temperature (exotherm seen on addition of HATU (25 °C - 32 °C)). The reaction was stirred at room temperature for 18 hours. Water (160 ml) was added (slight exotherm) followed by DCM (160 ml). The organic layer was separated and the aqueous layer extracted with dichloromethane (2 x 160 ml). The organics were combined and washed with 50% brine / water (320 ml), dried (sodium sulphate), filtered and the solvent removed *in vacuo* to give 37.5 g, brown oil, which was initially purified by silica chromatography eluting with 10% methanol / ethyl acetate. The enantiomers were separated by chiral chromatography using a Rainin preparative instrument (200ml heads) and column (Merck 50mm 20μm Chiralpak AD - No AD00SC-A1003) eluting with iso-Hexane/EtOH 50/50 to give the subtitled compound in 99% ee. (B.P. McDermott et al, Synlett, 2008, 6, p0875 - 0879)
$^1$H NMR (400.13 MHz, DMSO-$d_6$) δ ppm 0.95 (9H, s), 1.35 (9H, d, rotamers), 2.0 (1H, m), 2.2-2.3 (4H, m), 2.8-3.6 (11H, m), 4.6-4.75 (1H, m), 7.2-7.35 (5H, m).

**Step 5: Preparation of tert-butyl (2R)-4-tert-butyl-2-(piperazine-1-carbonyl) piperazine-1-carboxylate**

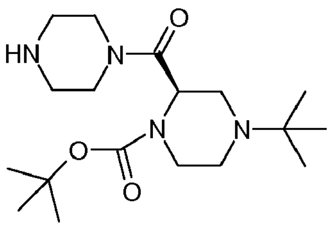

To a solution of tert-butyl (2R)-2-(4-benzylpiperazine-1-carbonyl)-4-tert-butylpiperazine-1-carboxylate (10 g) in ethanol (100 ml) was added 10% Palladium on carbon (50% wet, 2 ml), and the resulting mixture was hydrogenated at 5 barr at 35 °C for 18 hours. The reaction mixture was filtered and concentrated. The resulting gum was titrated with isohexane and filtered to give the titled compound (7.9 g) as a white solid.

**4. Preparation of N-(5-chloro-4-ethyl-1,3-thiazol-2-yl)piperazine-1-carboxamide**

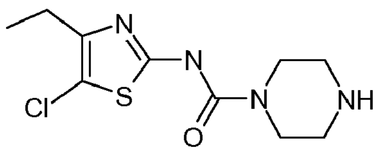

To a solution of tert-butyl piperazine-1-carboxylate (198 mg) in DCM (10 ml) was added TEA (444 μl) followed by phenyl N-(5-chloro-4-ethyl-1,3-thiazol-2-yl)carbamate (299 mg) and the resulting mixture was heated at 60 °C for 1 hour. The mixture was washed with water and the organics were dried ($MgSO_4$), filtered and concentrated under reduced pressure. The residue was purified by silica chromatography eluting with a gradient of EtOAc/isohexane (0 to 50%). The resulting product was dissolved in DCM (5 ml), TFA (2 ml) was added and the resulting mixture was stirred at room temperature for 2 hours. The reaction mixture was transferred to a SCX column and eluted with methanol, followed by 10% 7N $NH_3$ in methanol to afford the titled compound (90 mg). $^1H$ NMR (400.132 MHz, $CDCl_3$) δ ppm 1.2 (3H, t), 2.6 (2H, q), 2.9 (4H, m), 3.5 (4H, m). LCMS *M*/z(+) 275 (*M*+$H^+$).

In a similar manner, but using the appropriate carbamates the desired piperazine ureas were prepared.

**Pain models**

CCR2b Antagonist Behavioural Experimental Procedures

Induction of rat Spinal Nerve Ligated (SNL) model of neuropathic pain

Under isoflurane anesthesia, an incision is made dorsal to the lumbosacral plexus. The paraspinal muscles (left side) are separated from the spinous processes, the L5 and L6 spinal nerves are isolated and tightly ligated with (4-0 silk suture) distal to the dorsal root ganglion and prior to entrance into the sciatic nerve. The incision is closed and the skin is sealed with tissue adhesive. Rats are allowed to recover and then placed in cages with soft bedding. All experiments are conducted between postoperative day 7 to day 44.

Induction of rat Chronic Constriction Injury (CCI) model of neuropathic pain

Under isoflurane anesthesia, a small incision is made 0.5 cm below the pelvis. The biceps femoris and the gluteus superficialis (left side) are separated. The sciatic nerve is exposed, isolated and four loose ligatures (4-0 chromic gut) with 1 mm spacing are placed around it. The nerve is then placed back in its natural position, and the incision is sealed. Rats are allowed to recover and then placed in a cage with soft bedding. All experiments are conducted between postoperative day 7 to day 44.

Assessment of neuropathic-induced (SNL or CCI) heat hyperalgesia

In order to assess the degree of heat hyperalgesia, rats are individually placed in Plexiglas boxes on a glass surface (maintained at 30°C) of the paw thermal stimulator system (IITC Life Science, Woodland Hills, USA, Model 390, Series 8), and allowed to acclimatize for 30 minutes. A thermal stimulus, in the form of a radiant heat beam, is focused onto the plantar surface of the affected paw. In each test session, rats are tested twice approximately 5 minutes apart. Paw withdrawal latencies (PWLs) are calculated as the mean of the 2 values. An assay cut off is set at 20 seconds. In all experiments, animals are tested before drug administration to ensure stable heat-hyperalgesia baseline.

Assessment of neuropathic-induced (SNL or CCI) mechanical hyperalgesia

Mechanical hyperalgesia is assessed using the Ugo Basile analgesy-meter (Ugo Basile, Comerio, Italy). Animals are gently restrained, and a steadily increasing pressure is applied to the dorsal surface of a hind paw via a dome-shaped tip (diameter of 1 mm). The pressure requires to elicit paw withdrawal (PWT: paw withdrawal threshold) is determined. An assay cutoff is set at 295g. In all experiments, animals are tested before drug administration to ensure stable mechanical hyperalgesia baseline.

Drug treatment:

A study typically consists of 5 groups. One group is naive and serves as a baseline control. The other 4 groups undergo surgery to induce nerve injury. One of the groups serves as a vehicle control while the remaining 3 groups are treated with drug of increasing dose concentration. Test compound is administered orally and is tested for heat and/or mechanical hyperalgesia 240 minutes later. In all cases, the experimenter is blind to the treatment.

Bioanalysis:

Satellite animals are used for plasma and brain tissue collection. Animals are injected with drug but were not subjected to any testing. Blood and brain are collected by decapitation without anesthesia at the appropriate time point.

Data analysis

Statistical significance is determined using two-way RM ANOVA using raw data followed by a post-hoc Holm-Sidak t-test. The level of statistical significance is set at $p \leq 0.05$. GraphPad Prism® Version 4 is used for non-linear regression analysis. Raw data is converted using the following formula:

$$\% \text{ anti-hyperalgesia} = (\text{PWL or PWT(dosed)-PWL or PWT (vehicle)}) / (\text{PWL or PWT (naïve)-PWL or PWT (vehicle)}) \times 100.$$

Data is expressed as mean ±SEM. The 50% effective dose and blood plasma concentration are calculated from the best-fit curve using the variable slope sigmoidal equation model.

**hMCP-1 THP1 Receptor-binding assay**

i) Preparation of membrane fragments

THP1 cells were grown in RPMI (Sigma) supplemented with 10% foetal calf serum, 2 mM glutamine (Gibco), 100 units/ml Penicillin and 100 μg/ml Streptomycin (Invitrogen). Membrane fragments were prepared using cell lysis/differential centrifugation methods as described previously (Siciliano et al., 1990, J. Biol. Chem., 265, 19658). Protein concentration was estimated by BCA protein assay (Pierce, Rockford, Illinois) according to the manufacturer's instructions.

ii) Assay

$^{125}$I-labeled MCP-1 was prepared using Bolton and Hunter conjugation (Bolton et al., 1973, Biochem. J., 133, 529; Amersham International plc).

Test compounds were dissolved in DMSO and further diluted in assay buffer (50 mM HEPES, 1 mM $CaCl_2$, 5 mM $MgCl_2$, 1 mM EDTA, 0.03% BSA, pH 7.2) to give a range of concentrations starting with a top final concentration of up to10 μM. All incubations had a 100 μl final volume and a DMSO concentration of 1%. Incubations contained 250 pM $^{125}$I-labeled MCP-1 (GE Healthcare), 0.5 mg Scintillation proximity assay beads (GE Healthcare RPNQ001) and cell membranes containing 6 x $10^6$cells/ml equivalent. Non-specific binding was determined by the inclusion of 10 μM of a known CCR2b antagonist in the place of test compound. Total binding was determined in the presence of 1% DMSO without compound. Incubations were performed in sealed optiplates and kept at room temperature for 16 hours after which the plates were counted on a Packard TopCount (Packard TopCount™). Dose-response curves were generated using "in-house" data analysis package incorporating Origin software to determine $IC_{50}$ values. Percent inhibitions were calculated for single concentrations of compound by using the following formula 100-((compound binding minus non-specific binding)/(total binding minus non-specific binding) X 100).
Each compound set out in the Examples below was tested as above and shown to have an $IC_{50}$ value of better than 20 μM

| Example Number | CCR2 Binding $pIC_{50}$ |
|---|---|
| 1 | 8.51 |
| 2 | 8.59 |
| 3 | 8.55 |
| 4 | 8.17 |
| 5 | 8.7 |
| 6 | 8.74 |
| 7 | 8.07 |
| 8 | 8.14 |
| 9 | 8.59 |
| 10 | 8.17 |
| 11 | 8.64 |
| 12 | 8.74 |
| 13 | >9.06 |
| 14 | 8.56 |
| 15 | >9.35 |
| 16 | 8.9 |
| 17 | >9.35 |

## Claims

1. 4-[(2R)-4-tert-butylpiperazine-2-carbonyl]-N-(4-chloro-3-fluorophenyl) piperazine-1-carboxamide as a base or a pharmaceutically acceptable salt thereof.

2. 4-[(2R)-4-tert-butylpiperazine-2-carbonyl]-N-(4-chloro-3-fluorophenyl) piperazine-1-carboxamide.

3. A compound selected from

4-[(((2R)-4-tert-butylpiperazine-2-carbonyl]-N[3(trifluoromethyl)phenyl] piperazine-1-carboxamide;
4-[(((2R)-4-tert-butylpiperazine-2-carbonyl]-N-(3-chlorophenyl)piperazine-1-carboxamide;
4-[(((2R)-4-tert-butylpiperazine-2-carbonyl]-N-[3-(trifluoromethylsulfanyl)phenyl] piperazine-1-carboxamide;
N-(4-bromophenyl)-4-[(2R)-4-tert-butylpiperazine-2-carbonyl]piperazine-1-carboxamide;
4-[(((2R)-4-tert-butylpiperazine-2-carbonyl]-N-[4-fluoro-3(trifluoromethyl) phenyl] piperazine-1-carboxamide;
4-[(((2R)-4-tert-butylpiperazine-2-carbonyl]-N-[4-methyl-3-(trifluoromethyl) phenyl]piperazine-1-carboxamide;
4-[(((2R)-4-tert-butylpiperazine-2-carbonyl]-N-(3-chloro-4-fluorophenyl) piperazine-1-carboxamide;
4-[(((2R)-4-tert-butylpiperazine-2-carbonyl]-N-[4-cyano-3-(trifluoromethyl) phenyl]piperazine-1-carboxamide;
4-[(((2R)-4-tert-butylpiperazine-2-carbonyl]-N-(4-chloro-3-fluorophenyl) piperazine-1-carboxamide;
4-[(((2R)-4-tert-butylpiperazine-2-carbonyl]-N-[3-(1,1,2,2-tetrafluoroethoxy) phenyl]piperazine-1-carboxamide;
4-[(((2R)-4-tert-butylpiperazine-2-carbonyl]-N-(4-chloro-3-methylphenyl) piperazine-1-carboxamide;
4-[(((2R)-4-tert-butylpiperazine-2-carbonyl]-N-(3-phenylmethoxyphenyl) piperazine-1-carboxamide;
4-[((2R)-4-tert-butylpiperazine-2-carbonyl]-N-[3-chloro-4-(trifluoromethoxy) phenyl]piperazine-1-carboxamide;
N-(5-bromo-4-methyl-1,3-thiazol-2-yl)-4-[(2R)-4-tert-butylpiperazine-2-carbonyl]piperazine-1-carboxamide;
4-[(((2R)-4-tert-butylpiperazine-2-carbonyl]-N-(5-chloro-4-ethyl-1,3-thiazol-2-yl)piperazine-1-carboxamide;
4-[(((2R)-4-tert-butylpiperazine-2-carbonyl]-N-(5-chloro-4-methyl-1,3-thiazol-2-yl)piperazine-1-carboxamide;
and
4-[(((2R)-4-tert-butylpiperazine-2-carbonyl]-N-[5-chloro-4-(trifluoromethyl)-1,3-thiazol-2-yl]piperazine-1-carboxamide;
as a base or a pharmaceutically acceptable salt thereof.

4. A pharmaceutical composition comprising a therapeutically effective amount of a compound according to any one of claims 1 to 3 and at least one pharmaceutically acceptable excipients, carriers or diluents.

5. A compound according to any one of claims 1 to 3, as a base or a pharmaceutically acceptable salt thereof, for use as a medicament.

6. The use of a compound according to any one of claims 1 to 3 as a base or a pharmaceutically acceptable salt thereof, in the manufacture of a medicament for the therapy of pain.

7. The use according to claim 6, wherein said pain is neuropathic pain.

8. A method for the therapy of pain in a warm-blooded animal, comprising administering to said animal in need of such therapy a therapeutically effective amount of a compound according to any one of claims 1 to 3 as a base or a pharmaceutically acceptable salt thereof.

9. A method for the therapy of pain in a warm-blooded animal, comprising administering to said animal in need of such therapy a pharmaceutical composition according to claim 4.

10. The method according to claim 8 or 9, wherein said pain is neuropathic pain.

11. A compound according to claims 1-3 as a base or a pharmaceutically acceptable salt thereof, for the treatment of pain.

12. A compound according to claims 1-3 as a base or a pharmaceutically acceptable salt thereof, for the treatment of neuropathic pain.

Europäisches Patentamt
European Patent Office
Office européen des brevets

# EUROPEAN SEARCH REPORT

Application Number
EP 14 15 3398

**DOCUMENTS CONSIDERED TO BE RELEVANT**

| Category | Citation of document with indication, where appropriate, of relevant passages | Relevant to claim | CLASSIFICATION OF THE APPLICATION (IPC) |
|---|---|---|---|
| X | WO 2006/067401 A1 (ASTRAZENECA AB [SE]; ASTRAZENECA UK LTD [GB]; BOWER JUSTIN FAIRFIELD [) 29 June 2006 (2006-06-29) * page 1, lines 3 and 4; page 19, line 1; examples 211, 214, 222, 296, 333, 334 and 336; claims 1 and 9 * ----- | 1-12 | INV. C07D241/04 C07D417/12 A61K31/496 A61P25/00 |
| X | MCDERMOTT B P ET AL: "First example of s-BuLi/(-)-sparteine-mediated chiral deprotonation of a piperazine and proof of the sense of induction", SYNLETT, GEORG THIEME VERLAG, DE, vol. 2008, no. 6, 11 March 2008 (2008-03-11), pages 875-879, XP003026441, ISSN: 0936-5214, DOI: 10.1055/S-2008-1042905 * figure 5; compound 12 * ----- | 3,4 | |
| A,D | WO 2007/071952 A1 (ASTRAZENECA AB [SE]; ASTRAZENECA UK LTD [GB]; BOWER JUSTIN FAIRFIELD [) 28 June 2007 (2007-06-28) * claims 1, 6; examples 85-89 * ----- | 1-12 | TECHNICAL FIELDS SEARCHED (IPC) C07D A61K A61P |

The present search report has been drawn up for all claims

1

| Place of search | Date of completion of the search | Examiner |
|---|---|---|
| Munich | 28 March 2014 | Moriggi, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 14 15 3398

This annex lists the patent family members relating to the patent documents cited in the above-mentioned European search report.
The members are as contained in the European Patent Office EDP file on
The European Patent Office is in no way liable for these particulars which are merely given for the purpose of information.

28-03-2014

| Patent document cited in search report | Publication date | Patent family member(s) | Publication date |
|---|---|---|---|
| WO 2006067401 A1 | 29-06-2006 | AR 053109 A1 | 25-04-2007 |
| | | AU 2005317928 A1 | 29-06-2006 |
| | | AU 2010212346 A1 | 09-09-2010 |
| | | BR PI0519288 A2 | 06-01-2009 |
| | | CA 2589748 A1 | 29-06-2006 |
| | | EP 1831164 A1 | 12-09-2007 |
| | | HK 1110868 A1 | 13-12-2013 |
| | | IL 183665 A | 29-02-2012 |
| | | JP 5118975 B2 | 16-01-2013 |
| | | JP 2008525397 A | 17-07-2008 |
| | | JP 2012214507 A | 08-11-2012 |
| | | KR 20070091677 A | 11-09-2007 |
| | | MY 147400 A | 30-11-2012 |
| | | NZ 555770 A | 24-12-2010 |
| | | US 2009099156 A1 | 16-04-2009 |
| | | US 2011136820 A1 | 09-06-2011 |
| | | US 2012264762 A1 | 18-10-2012 |
| | | US 2014038978 A1 | 06-02-2014 |
| | | UY 29312 A1 | 31-07-2006 |
| | | WO 2006067401 A1 | 29-06-2006 |
| WO 2007071952 A1 | 28-06-2007 | CN 101384582 A | 11-03-2009 |
| | | EP 1966187 A1 | 10-09-2008 |
| | | JP 2009520781 A | 28-05-2009 |
| | | US 2008287453 A1 | 20-11-2008 |
| | | WO 2007071952 A1 | 28-06-2007 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2006067401 A
- WO 2004110376 A2, Zhang
- WO 2004110376 B, White
- WO 9722596 A
- WO 9730035 A
- WO 9732856 A
- WO 9813354 A
- WO 9902166 A
- WO 0040529 A
- WO 0041669 A
- WO 0192224 A
- WO 0204434 A
- WO 0208213 A


### Non-patent literature cited in the description


- **GREENE ; WUTS.** Protective Groups in Organic Synthesis. 1999
- **CORWIN HANSCH.** Comprehensive Medicinal Chemistry. Pergamon Press, 1990, vol. 5
- **B.P. MCDERMOTT et al.** *Synlett,* 2008, vol. 6, 0875-0879
- **SICILIANO et al.** *J. Biol. Chem.,* 1990, vol. 265, 19658
- **BOLTON et al.** *Biochem. J.,* 1973, vol. 133, 529